# EUROPEAN PATENT APPLICATION

(11) **EP 2 368 876 A1**
(43) Date of publication of application: **28.09.2011**
(21) Application number: 10305207.2
(22) Date of filing: 01.03.2010
(51) Int. Cl.: C07D 205/04, C07D 207/08, C07D 211/56, C07D 211/58, C07D 243/08, C07D 295/08, C07D 401/02, C07D 401/12, C07D 403/12, C07D 405/12, C07D 413/12, C07D 487/08, A61K 31/4418, A61K 31/40, A61K 31/444, A61K 31/551, A61K 31/4439, A61K 31/4704, A61P 9/00, A61P 21/00, A61P 13/12, A61P 9/10, A61P 9/04, A61P 35/00, A61P 11/00, A61P 25/04, A61P 31/00, A61P 7/00

(54) **Derivatives of aminoindanes, their preparation and their application in therapeutics**

(71) Applicant: SANOFI, 75013 Paris (FR)
(72) Inventor: The designation of the inventor has not yet been filed
(74) Representative: Jacobson, Claude

(57) **Abstract**

The instant invention relates to derivatives of formula (I) 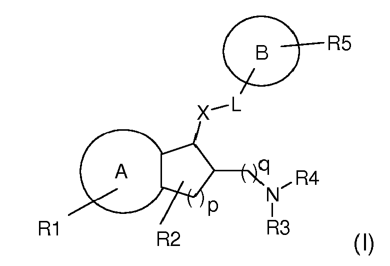 and their application in therapeutics.

## Description

The instant invention relates to derivatives of aminoindanes, to their preparation and to their application in therapeutics.

Transient receptor potential cation channel, subfamily C, member 6, also known as TRPC6, is a human gene encoding a protein of the same name. TRPC6 has been associated with fibrotic disorders, such as focal segmental glomerulosclerosis (a) Winn et al, Science 2005, 308, 1801-1804. b) Hsu et al., Biochim. Biophys. Acta, Molec. Basis of Disease 2007, 1772, 928-936. c) Kriz, Trends Molec. Med. 2005, 11, 527-530. d) Winn et al, J. Amer.Soc.Nephrol. 2005, 17, 378-387), skeletal muscle dysfunction (Millay et al., PNAS 2009, 106, 19023 -19028), renal failure, atherosclerosis, heart failure (Kuwahara et al., J. Clin. Invest. 2006, 116, 3114-26), cancer (e.g. oesophageal cancer, breast cancer) (a) Aydar et al., Cancer Cell Int. 2009, 9, 23. b) Cai et al., Int. J. Cancer. 2009, 125, 2281-2287. c) Shi et al., Gut 2009, 58, 1443-1450), chronic obstructive pulmonary disease (Sel et al., Clin. Exp. Allergy. 2008, 38, 1548-1558), pain (Alessandri-Haber et al., J. Neurosci. 2009, 29, 6217-6228), pulmonary hypertension (Yu et al., Circulation 2009, 119, 2313-2322), ischemic stroke, myocardial infarction (Varga-Szabo et al., J. Thromb. and Haemost. 2009, 7, 1057-1066), inflammation or peripheral arterial occlusive disease.

It is thus desirable to provide novel TRPC6 inhibitors for the prevention or treatment of these pathologies.

The compounds according to the instant invention respond to the general formula (I) : 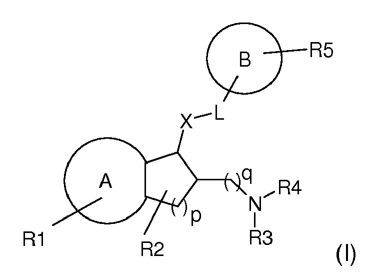 in which
- A: is a 6 to 10 membered aryl radical or a 5 to 10 membered heteroaryl radical, where the aryl and heteroaryl radical may be mono- or bicyclic, and the heteroaryl radical may comprise one or more heteroatoms selected from the group of
nitrogen, oxygen and sulfur;
where one or more hydrogen atoms in said mono- or bicyclic aryl or heteroaryl radicals may be replaced by substituents R1 which are selected independently of one another from the group of H, F, Cl, Br, I, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₄-C₂₀)-cycloalkylalkyloxy, (C₁-C₁₀)-alkoxy, (C₁-C₁₀)-alkylthio, (C₆-C,₄)-aryl, (C₂-C₁₃)-heteroaryl, -CN, -OH, -NR13R14, -C(O)R12, -SF₅, -S(O)ₙR12, -C(O)OR12, -C(O)NR13R14, -S(O)ₙNR13R14;
   where two adjacent radicals R1 may also form a saturated or partly unsaturated (C₅-C₁₀)-cycloalkyl radical or a saturated or partly unsaturated (C₂-C₉)-cycloheteroalkyl radicals, where the cycloheteroalkyl radical may comprise 1, 2 or 3 nitrogen, 1 or 2 oxygen, 1 or 2 sulfur, 1 or 2 nitrogen and 1 oxygen or 1 sulfur atom;
      where said alkyl, alkenyl, alkynyl, cycloalkyl, cycloalkylalkyl, cycloalkylalkyloxy, cycloheteroalkyl, alkoxy, and alkylthio radicals may be substituted independently of one another one or more times by F, OH or (C₁-C₁₀)-alkoxy;
- B: is a mono- or fused bicyclic radical selected from the group of
6 to 10 membered aryl radicals,
of 5 to 10 membered heteroaryl radicals,
of 3 to 10 membered cycloalkyl radicals,
of 9 to 14 membered cycloalkylaryl radicals,
of 8 to 14 membered cycloalkylheteroaryl radicals,
of 3 to 10 membered cycloheteroalkyl radicals,
of 9 to 14 membered cycloheteroalkylaryl radicals and
of 8 to 14 membered cycloheteroalkylheteroaryl radicals,
where the cycloalkyl or cycloheteroalkyl units may be saturated or partly unsaturated, and where the heterocyclic groups may comprise one or more heteroatoms selected from the group of nitrogen, oxygen and sulfur;
where one or more hydrogen atoms in the radicals B may be replaced by substituents R5 which are selected independently of one another from the group of (C₁-C₁₀)-alkyl radicals, of (C₂-C₁₀)-alkenyl radicals, of (C₂-C₁₀)-alkynyl radicals, of (C₁-C₁₀)-alkoxy radicals, of (C₁-C₁₀)-alkylthio radicals, of (C₃-C₁₄)-cycloalkyl radicals, of (C₄-C₂₀)-cycloalkylalkyl radicals, of (C₄-C₂₀)-cycloalkylalkyloxy, of (C₂-C₁₉)-cycloheteroalkyl radicals, of (C₃-C₁₉)-cycloheteroalkylalkyl radicals, of (C₃-C₁₁)-cycloalkyloxy radicals, of (C₂-C₁₁)-cycloheteroalkyloxy radicals, of (C₆-C₁₀)-aryl radicals, of (C₁-C₉)-heteroaryl radicals, of (C₉-C₁₄)-cycloalkylaryl radicals, of (C₅-C₁₃)-cycloalkylheteroaryl radicals, (C₇-C₁₃)-cycloheteroalkylaryl radicals, (C₄-C₁₂)-cycloheteroalkylheteroaryl radicals, where
   the cycloalkyl and cycloheteroalkyl units may be saturated or partly unsaturated, and where one or more hydrogen atoms in said radicals R5 may be replaced by further radicals which are selected independently of one another from the group of R11 radicals,
it is further possible for R5 to be one or more radicals which are selected independently of one another from the group of H, OH, (=O), NH₂, F, Cl, Br, I, CN, NO₂, -NR17R18, -NR16COR17, -NR16COOR17, -NR16CONR17R18, -NR16-S(O)₂-R17, -NR16-S(O)₂-NR17R18, -COOR16, -COR16; -CO(NR17R18), S(O)ₙR16, -S(O)₂NR17R18,
   where R16, R17 and R18 independently of one another for a radical selected from the group of H, (C₂-C₁₉)-cycloheteroalkyl, (C₃-C₁₄)-cycloalkyl, (C₆-C₁₀)-aryl, (C₁-C₁₀)-alkyl radicals,
      all of which may be substituted independently of one another by OH, (=O), F, Cl, Br, I, CN, NO₂, -NR13R14, -NR13COR12, -NR13COOR12, -NR12CONR13R14, -NR13-S(O)₂-R12, -NR12-S(O)₂-R13R14, -COOR12, -COR12; -CO(NR13R14), -S(O)ₙR12, -S(O)₂NR13R14, (C₃-C₁₄)-cycloalkyl, (C₄-C₂₀)-cycloalkylalkyl, (C₂-C₁₉)-cycloheteroalkyl, (C₃-C₁₉)-cycloheteroalkylalkyl, (C₆-C₁₀)-aryl and (C₁-C₉)-heteroaryl,
   and where R17 and R18 can form together with the nitrogen to which they are bonded a 4-7 membered, saturated, unsaturated or partly unsaturated heterocycle having 1 to 13 carbon atoms which may additionally comprise one or more heteroatoms from the list -O-, -S(O)ₙ-, =N- and -NR15-,
      where the heterocycle formed may be substituted independently of one another one or more times by F, OH, (=O), NH₂, NH(C₁-C₄)alkyl, N((C₁-C₄)alkyl)₂, CN or (C₁-C₁₀)-alkoxy, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₂₀)-cycloheteroalkyl, (C₃-C₁₉)-cycloheteroalkylalkyl, each of which may in turn carry independently of one another one or more radicals F, OH, (=O), NH₂, NH(C₁-C₄)alkyl, N((C₁-C₄)alkyl)₂, CN or (C₁-C₁₀)-alkoxy;
- L: is a covalent bond or an alkylene bridge having 1 to 10 carbon atoms, which may carry independently of one another one or more substituents from the group of radicals (C₁-C₁₀)-alkyl, (C₃-C₁₄)-cycloalkyl, (C₄-C₂₀)-cycloalkylalkyl radical, -COR12, -CO(NR13R14), S(O)ₙR12, -S(O)₂NR13R14, (=O) and F; where the alkyl, cycloalkyl and cycloalkyl radicals may be substituted one or more times by F;
- X: is a group -N(R6)-, -O-, -S(O)ₙ-, or alkylene having 1 to 5 carbon atoms, where R6 may be hydrogen or may be (C₁-C₁₀)-alkyl, (C₃-C₁₄)-cycloalkyl, (C₄-C₂₀)-cycloalkylalkyl radical, all of which may be substituted independently of one another one or more times by F, or R6 may be -COR12; -CO(NR13R14), S(O)ₙR12, -S(O)₂NR13R14;
- R2: is absent or is one or more substituents which may be selected independently of one another from the group of F, (C₁-C₁₀)-alkyl and (C₁-C₁₀)-alkoxy radical, where the alkyl and alkoxy radicals may be substituted independently of one another one or more times by F;
- R3 and R4: are independently of one another a hydrogen radical or a radical which is selected from the group of (C₁-C₁₀)-alkyl radicals, of (C₂-C₁₀)-alkenyl radicals, of (C₂-C₁₀)-alkynyl radicals, of (C₃-C₁₄)-cycloalkyl radicals, of (C₄-C₂₀)-cycloalkylalkyl radicals, of (C₂-C₁₉)-cycloheteroalkyl radicals, of (C₃-C₁₉)-cycloheteroalkylalkyl radicals, of (C₆-C₁₀)-aryl radicals, of (C₇-C₂₀)-arylalkyl radicals, of (C₁-C₉)-heteroaryl radicals, of (C₂-C₁₉)-heteroarylalkyl radicals, where
the radicals R3 and R4 may be substituted independently of one another one or more times by a radical from the group of OH, NH₂, (=O), F, Cl, Br, I, CN, NO₂, -NR13R14, -NR13COR12, -NR13COOR12, -NR12CONR13R14, -NR13-S(O)₂-R12, -NR13-S(O)₂-NR13R14, -COOR12, -COR12; -CO(NR13R14), S(O)ₙR12, -S(O)₂R13R14, or
- R3 and R4: form together with the nitrogen to which they are bonded a 4-10 membered, saturated, unsaturated or partly unsaturated heterocycle which may additionally comprise one or more heteroatoms from the list -O-, -S(O)ₙ-, =N- and -NR8-, where
the heterocyclic radicals may be substituted independently of one another one or more times by radicals selected from the group of R7 and R9, and where
the heterocyclic radicals may be bridged by a bond, by a saturated or unsaturated (C₁-C₁₀)-alkyl or (C₁-C₉)-heteroalkyl chain or by -NR15-, -O-, -S-, and where
   the alkyl and heteroalkyl chains may also form a spirocyclic ring system with the ring system formed by R3 and R4, where the alkyl and heteroalkyl bridges may be substituted independently of one another one or more times by radicals selected from the group of R7 and R9,
and where
R8 in the group NR8 may form with the ring which R3 and R4 may form a further saturated, unsaturated or partly unsaturated heterocycle which may be substituted independently of one another one or more times by radicals selected from the group of R7 and R9, and may additionally comprise one or more heteroatoms from the list -O-, -S(O)ₙ-, -N= and -NR19-;
- R7: are a (C₁-C₁₀)-alkyl radical or (C₁-C₁₄)-cycloalkyl radical, where the alkyl radical may be substituted independently of one another one or more times by R9;
- R8: is an H, a (C₁-C₁₀)-alkyl radical or (C₁-C₁₄)-cycloalkyl radical, COR12, -CO(NR13R14), S(O)ₙR12, -S(O)₂NR13R14 where the alkyl radical may be substituted independently of one another one or more times by R10;
- R9: is a radical selected from the group of OH, (=O), F, Cl, Br, I, CN, NO₂ -NR13R14, -NR13COR12, -NR13COOR12, -NR12CONR13R14, -NR13-S(O)₂-R12, -NR13-S(O)₂-NR13R14, -COOR12, -COR12, -CO(NR13R14), S(O)ₙR12, -S(O)₂NR13R1 (C₃-C₁₄)-cycloalkyl, (C₄-C₂₀)-cycloalkylalkyl, (C₁-C₁₀)-alkoxy, (C₂-C₁₈)-cycloheteroalkyl, (C₃-C₁₉)-cycloheteroalkylalkyl, (C₆-C₁₀)-aryl radicals, of (C₁-C₉)-heteroaryl radicals;
- R10: is a radical selected from the group of F, OH, CN, (C₁-C₁₀)-alkoxy, (C₁-C₁₀)- alkylthio, NO₂, -NR13R14, -NR13COR12, -NR13COOR12, -NR13CONR13R14, -NR13-S(O)₂-R12, -NR12-S(O)₂-NR13R14, -COOR12, -COR12, -CO(NR13R14), S(O)ₙR12, -S(O)₂NR13R14;
- R11: is a radical selected from the group of (C₁-C₁₀)-alkyl, (C₂-C₁₀)-alkenyl, (C₂-C₁₀)-alkynyl, (C₁-C₁₀)-alkoxy, (C₁-C₂₀)-alkylthio, (C₃-C₁₄)-cycloalkyl, (C₄-C₁₀)-cycloalkylalkyl, (C₂-C₁₃)-cycloheteroalkyl, (C₄-C₁₉)-cycloheteroalkylalkyl, (C₃-C₁₄)-cycloalkyloxy, (C₂-C₁₃)-cycloheteroalkyloxy,
all of which may be substituted independently of one another one or more times by R10;
(=O), Cl, Br, I and R10;
- R12, R13 and R14: may independently of one another be H, (C₁-C₁₀)-alkyl, (C₂-C₁₀)-alkenyl, (C₂-C₁₀)-alkynyl, (C₃-C₁₄)-cycloalkyl, (C₄-C₁₀)-cycloalkylalkyl, (C₂-C₁₃)-cycloheteroalkyl, (C₃-C₁₉)-cycloheteroalkylalkyl, (C₆-C₁₀)-aryl, each of which may be substituted independently of one another one or more times by F, OH, (=O), NH₂, NH(C₁-C₄)alkyl, N((C₁-C₄)alkyl)₂, CN or (C₁-C₁₀)-alkoxy;
or where R13 and R14 may form together with the nitrogen to which they are bonded a 4-7 membered, saturated, unsaturated or partly unsaturated heterocycle having 1 to 13 carbon atoms, which may additionally comprise one or more heteroatoms from the list -O-, -S(O)ₙ-, =N- and -NR15-, where
the formed heterocycle may be substituted independently of one another one or more times by F, OH, (=O), NH₂, NH(C₁-C₄)alkyl, N((C₁-C₄)alkyl)₂, CN or (C₁-C₁₀)-alkoxy, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₂₀)-cycloheteroalkyl, (C₃-C₁₉)-cycloheteroalkylalkyl, each of which may in turn carry independently of one another one or more radicals F, OH, (=O), NH₂, NH(C₁-C₄)alkyl, N((C₁-C₄)alkyl)₂, CN or (C₁-C₁₀)-alkoxy;
- R15: is a radical selected from the group of H, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₁₃)-cycloheteroalkyl, (C₃-C₁₉)-cycloheteroalkylalkyl, each of which may be substituted independently of one another one or more times by F, OH, CN or (C₁-C₁₀)-alkoxy;
- R19: is an H, a (C₁-C₁₀)-alkyl radical or (C₁-C₁₄)-cycloalkyl radical, COR12, -CO(NR13R14), S(O)ₙR12, -S(O)₂NR13R14, where the alkyl radical may be substituted independently of one another one or more times by R10;
and in which
- n: is 0, 1 or 2;
- p: is 1 or 2 and
- q: is 0 or 1,
and the pharmaceutically acceptable salts thereof,
and in which
i) in the case where A is phenyl, B is phenyl or benzodioxolanyl, X is -O- or -S-, L is a bond and R3 and R4 are H, (C₁-C₁₀)-alkyl, (C₃-C₁₄)-cycloalkyl, (C₇-C₂₀)-arylalkyl or R3 and R4 together are an unsubstituted pyrrolidinyl, morpholinyl, piperidinyl or piperazinyl radical or 4-methylpiperazinyl radical, at least one R5 radical which is not a (C₁-C₁₀)-alkyl, (C₁-C₁₀)-alkoxy, OH, CF₃, F, Cl, Br or I radical must be present,
ii) in the case where A is phenyl, X is -O-, -S- or -NH- and R3 and R4 are a (C₁-C₁₀)-alkyl, (C₃-C₁₄)-cycloalkyl or a (C₄-C₂₀)-cycloalkylalkyl radical, at least one R5 radical which is not an F, Cl, Br, I, (C₁-C₄)-alkyl, (C₁-C₄)-alkoxy, CF₃, OCF₃, CN, NO₂ NH₂, -NH((C₁-C₁₀)-alkyl), -N((C₁-C₁₀)-alkyl)₂, unsubstituted or substituted benzoyl or an unsubstituted or substituted phenyl-(CH₂)ᵣ-Y-(CH₂)ₛ- radical, with Y being a bond or an oxygen and r and s being 0 to 4, where r+s is not greater than 4, must be present.

In one embodiment, compounds of the formula I and the pharmaceutically acceptable salts thereof are preferred wherein:
L is a covalent bond;
X is a group -O-;
and q is 0.

The compounds of formula (I) can comprise one or more asymmetric carbon atoms. They can therefore exist in the form of enantiomers or diastereoisomers. These enantiomers and diastereoisomers, as well as their mixtures, including racemic mixtures, form part of the invention.

The compounds of formula (I) can be provided in the form of a free base or in the form of addition salts with acids, which also form part of the invention.

These salts are advantageously prepared with pharmaceutically acceptable acids, but salts with other acids, useful for example for the purification or for the isolation of the compounds of formula (I), also form part of the invention.

The compounds of formula (I) can also be provided in the form of an hydrate or of a solvate, i.e. in the form of associations or combinations with one or more water or solvent molecules. Such hydrates and solvates also form part of the invention.

According to the present invention, the terms below have the following meanings:
- a halogen atom corresponds to a fluorine, chlorine, bromine or iodine atom;
- (C₁-C₁₀)-Alkyl radicals may in the context of the present invention be straight-chain or branched. This also applies when they carry substituents or occur as substituents of other radicals, for example in fluoroalkyl radicals or alkoxy radicals. Examples of alkyl radicals are methyl, ethyl, n-propyl, isopropyl (= 1-methylethyl), n-butyl, isobutyl (= 2-methylpropyl), sec-butyl (= 1-methylpropyl), tert-butyl (= 1,1-dimethylethyl), n-pentyl, isopentyl, tert-pentyl, neopentyl and hexyl. Preferred alkyl radicals are methyl, ethyl, n-propyl, isopropyl, n-butyl and tert-butyl.
- (C₃-C₁₄)-Cycloalkyl radicals in the context of the present invention may be saturated or partly unsaturated. This also applies when they carry substituents or occur as substituents of other radicals. Cycloalkyl radicals having 3, 4, 5, 6, 7 or 8 carbon atoms are preferred. Examples of cycloalkyl radicals are cyclopropyl, cyclobutyl, cyclopentyl, cyclohexyl, cycloheptyl and cyclooctyl a fluoroalkyl group corresponds to an alkyl group, wherein one or more hydrogen atoms have been substituted by fluorine atoms;
- (C₂-C₁₉)-Cycloheteroalkyl radicals in the context of the present invention may be saturated or partly unsaturated. This also applies when they carry substituents or occur as substituents of other radicals. The cycloheteroalkyl radicals preferably have heteroatoms selected from the group of nitrogen, oxygen and sulfur. Cycloheteroalkyl radicals having 2, 3, 4, 5, 6, 7, 8 or 9 carbon atoms are preferred, it being possible for 1 or 2 nitrogen atoms, 1 or 2 oxygen atoms, 1 or 2 sulfur atoms, 1 nitrogen and 1 oxygen atom or 1 sulfur atom or 1 oxygen and 1 sulfur atom to be present as heteroatoms. The cycloheteroalkyl radicals can be attached by any position. Examples of such heterocycles are selected from the group of oxiranyl, thiiranyl, aziridinyl, oxetanyl, thietanyl, azetidinyl, diazetidinyl, pyrrolidinyl, dihydropyrrolyl, dihydroimidazolyl, dihydropyrazolyl, tetrahydropyrazolyl, oxolanyl, dihydrofuranyl, dioxolanyl, thiolanyl, dihydrothiophenyl, oxazolanyl, dihydrooxazolyl, isooxazolanyl, dihydroisooxazolyl, thiazolidinyl, dihydrothiazolyl, isothiazolidinyl, dihydroisothiazolyl, oxathiolidinyl, *2H*-pyranyl, *4H*-pyranyl, tetrahydropyranyl, *2H*-thiopyranyl, *4H*-thiapyranyl, tetrahydrothiopyranyl, piperidinyl, di-, tetrahydropyridyl, piperazinyl, di-, tetrahydropyrazinyl, di-, tetra-, hexahydropyridazinyl, di-, tetra-, hexahydropyrimidinyl, morpholinyl, thiomorpholinyl, azepanyl, thiepanyl and oxepinyl, it also being possible for two of these heterocyclic rings to form a saturated or partly unsaturated fused bicyclic ring system. Examples of such bicyclic ring systems are octahydropyrrolo[1,2a]pyrazinyl, octahydropyrrolo[3,4b]pyrrolyl, hexahydropyrrolo[3,4-c]pyrrolyl- and octahydropyrrolo[3,4-c]pyrrolyl. Prefered cycloheteroalkyl are azetidinyl, pyrrolidinyl, piperidinyl, homopiperazine or 2,5-diazabicyclo[2.2.1]heptane.
- (C₂-C₁₀)-Alkenyl radicals in the context of the present invention may likewise be straight-chain or branched. This also applies when they carry substituents or occur as substituents of other radicals. Examples of alkenyl radicals are ethenyl, propenyl and butenyl.
- (C₂-C₁₀)-Alkynyl radicals in the context of the present invention may likewise be straight-chain or branched. This also applies when they carry substituents or occur as substituents of other radicals. Examples of alkynyl radicals are ethynyl, propynyl and butynyl.an alkoxy group corresponds to an -O-alkyl group, wherein the alkyl group is as defined above;
- Examples of preferred (C₆-C₁₀)-aryl radicals are phenyl and naphthyl. This also applies when they carry substituents or occur as substituents of other radicals.

(C₁-C₉)-Heteroaryl radicals are aromatic ring compounds in which one or more ring atoms are oxygen atoms, sulfur atoms or nitrogen atoms, e.g. 1, 2 or 3 nitrogen atoms, 1 or 2 oxygen atoms, 1 or 2 sulfur atoms or a combination of various heteroatoms. This also applies when they carry substituents or occur as substituents of other radicals. The heteroaryl radicals may be attached by all positions. Heteroaryl means for example furanyl, thiophenyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl and cinnolinyl.

Preferred heteroaryl radicals are 2- or 3-thiophenyl, 2- or 3-furyl, 1-, 2- or 3- pyrrolyl, 1-, 2-, 4- or 5-imidazolyl, 1-, 3-, 4- or 5-pyrazolyl, 1,2,3-triazol-1-, -4- or -5-yl, 1,2,4-triazol-1-, -3- or -5-yl, 1- or 5-tetrazolyl, 2-, 4- or 5-oxazolyl, 3-, 4- or 5-isoxazolyl, 1,2,3-oxadiazol-4- or -5-yl, 1,2,4-oxadiazol-3- or -5-yl, 1,3,4-oxadiazol-2-yl or -5-yl, 2-, 4- or 5-thiazolyl, 3-, 4- or 5-isothiazolyl, 1,3,4-thiadiazol-2- or -5-yl, 1,2,4-thiadiazol-3- or -5-yl, 1,2,3-thiadiazol-4- or -5-yl, 2-, 3- or 4-pyridyl, 2-, 4-, 5- or 6-pyrimidinyl, 3- or 4-pyridazinyl, pyrazinyl, 1-, 2-, 3-, 4-, 5-, 6- or 7-indolyl, 1-, 2-, 4- or 5-benzimidazolyl, 1-, 3-, 4-, 5-, 6- or 7-indazolyl, 2-, 3-, 4-, 5-, 6-, 7- or 8-quinolyl, 1-, 3-, 4-, 5-, 6-, 7- or 8-isoquinolyl, 2-, 4-, 5-, 6-, 7- or 8-quinazolinyl, 3-, 4-, 5-, 6-, 7- or 8-cinnolinyl, 2-, 3-, 5-, 6-, 7- or 8-quinoxalinyl, 1-, 4-, 5-, 6-, 7- or 8-phthalazinyl..

Particularly preferred heteroaryl radicals are pyrazolyl, isoxasolyl, benzotriazolyl;
- (C₉-C1₄)-Cycloalkylaryl radicals are preferably selected from the group of fused ring systems having a cycloalkyl ring and an aryl ring, in particular a phenyl ring. Particularly preferred cycloalkylaryl radicals are indenyl, dihydronaphthyl, tetrahydronaphthyl and indanyl.
- (C₅-C₁₃)-Cycloalkylheteroaryl radicals are preferably selected from the group of fused ring systems having a cycloalkyl ring and a heteroaryl ring.
- (C₇-C₁₃)-Cycloheteroalkylaryl radicals are preferably fused ring systems having a cycloheteroalkyl ring and an aryl ring, in particular a phenyl ring. Preferred cycloheteroalkylaryl radicals are benzodihydrothiophenyl, benzothiolanyl, benzodihydrofuranyl, benzooxolanyl, benzodioxolanyl, benzodihydropyrrolyl, benzodihydroimidazolyl, benzodihydropyrazolyl, benzodihydrotriazolyl, benzopiperazinyl, benzodihydrothiazolyl, benzomorpholinyl benzodihydrooxazolyl, dihydroquinolinyl, dihydroisoquinolinyl, tetrahydroisoquinolinyl and tetrahydroquinolinyl. Particularly preferred are cycloheteroalkylaryl radicals of formula: 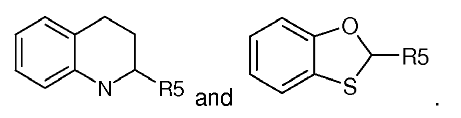
- (C₄-C₁₂)-Cycloheteroalkylheteroaryl radicals are preferably selected from the group of fused ring systems having a cycloheteroalkyl ring and a heteroaryl ring.
- A cycloheteroalkyl corresponds to a cyclic group comprising between 3 and 19 carbon atoms and between 1 and 3 heteroatoms, preferably nitrogen atoms.

The compounds of the invention advantageously respond to the general formula (II) : 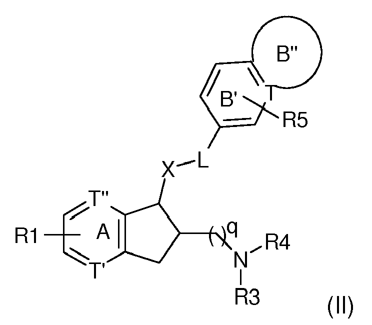 in which:
- A: is a 6 membered aryl radical or a 6 membered heteroaryl radical, where T' and T" are independently N or CR1;
where one or more hydrogen atoms in said aryl or heteroaryl radicals may be replaced by substituents R1 which are selected independently of one another from the group of H, F, Cl, Br, I, (C₁-C₁₀)-alkyl-, (C₁-C₁₀)-alkoxy-, -CN, -OH, -S(O)ₙR12, -;
   where said alkyl and alkoxy radicals may be substituted independently of one another one or more times by F and R12 is H or (C₁-C₁₀)-alkyl, optionally substituted one or more times by F;
- B': is a 6 membered aryl radical or a 6 membered heteroaryl radical, where T is N or CR5;
- B": is absent or is radical chosen from a (C₆-C₁₀)-aryl radical and a (C₃-C₆)-cycloheteroalkyl radical comprising one ore more heteroatoms chosen from O, N and S,
where at least one hydrogen atoms in the radicals B' and/or B" is replaced by substituents R5 which are selected independently of one another from the group of (C₁-C₁₀)-alkyl radicals, of (C₁-C₁₀)-alkoxy radicals, of (C₃-C₁₄)-cycloalkyl radicals, of (C₁-C₉)-heteroaryl radicals, where
   the cycloalkyl units may be saturated or partly unsaturated, and where one or more hydrogen atoms in said radicals R5 may be replaced by further radicals which are selected independently of one another from the group of F, Cl, Br and I,
it is further possible for R5 to be one or more radicals which are selected independently of one another from the group of OH, F, Cl, Br, I, CN, NO₂ -COOR16, -CO(NR17R18),
   where R16, R17 and R18 independently of one another for a radical selected from the group of H and (C₁-C₁₀)-alkyl radicals, which may be substituted independently of one another by F, Cl, Br, I,
- L: is a covalent bond or a methylene bridge;
- X: is a group -O- or -S(O)ₙ-;
- R3 and R4: are independently of one another a hydrogen radical, a (C₁-C₁₀)-alkyl radical or a (C₃-C₁₉)-cycloheteroalkyl radical comprising at least one nitrogen atom, or
- R3 and R4: form together with the nitrogen to which they are bonded a 4-7 membered, saturated mono-or bi-cycloheteroalkyl radical which may additionally comprise one or more -NR8- heteroatom, where
the cycloheteroalkyl radical may be substituted independently of one another one time by a radical-(CR20R21)ᵣ-NR22R23, in which r is 0 or 1 and R20, R21, R22 and R23 are independently of one another H or a (C₁-C₁₀)-alkyl radical optionally substituted one or more times by F, Cl, Br and/or I,
- R8: is an H or a (C₁-C₁₀)-alkyl radical;
and in which
- n: is 0, 1 or 2; and
- q: is 0 or 1,
in the form of a free base or of an addition salt with an acid, as well as in the form of an hydrate or of a solvate.

The compounds of the invention advantageously respond to the general formula (III) : 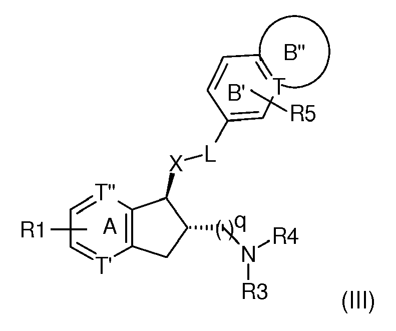 in which A, B', B", T, T', T", R1, R3, R4, R5, X, L and q are as defined above for compounds of formula (II), in the form of a free base or of an addition salt with an acid, as well as in the form of an hydrate or of a solvate.

Among the compounds of formula (I) according to the instant invention, the following compounds may be cited, in the same order as for the compounds depicted in the table hereafter, illustrating some examples of compounds:
(3R)-1-{(1R,2S)-5-chloro-1-[(2-chloro-5-fluorobenzyl)oxy]-2,3-dihydro-1H-inden-2-yl}piperidin-3-amine
(3S)-1-[(1R,2S)-5-chloro-1-{[2-fluoro-3-(trifluoromethyl)benzyl]oxy}-2,3-dihydro-1H-inden-2-yl]piperidin-3-amine
1-({(1S,2R)-1-[3-(trifluoromethoxy)phenoxy]-2,3-dihydro-1H-inden-2-yl}methyl)pyrrolidine
1-({(1S,2R)-1-[3-(trifluoromethoxy)phenoxy]-2,3-dihydro-1H-inden-2-yl}methyl)piperidine
1-[1-(2-cyclopentylphenoxy)-2,3-dihydro-1H-inden-2-yl]piperidin-4-amine
N-{(1-[(6-chloropyridin-3-yl)oxy]-2,3-dihydro-1H-inden-2-yl)-N-methylpiperidin-3-amine
(3R)-1-{(1R,2R)-1-[2-(2H-benzotriazol-2-yl)-4-methylphenoxy]-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)-3-chlorobenzonitrile
(3R)-1-[(1R,2R)-4,6-dichloro-1-(4-fluorophenoxy)-2,3-dihydro-1H-inden-2-yl]piperidin-3-amine
4-({(1R,2R)-2-[(3R)-3-aminopiperidin-1-yl]-4,6-dichloro-2,3-dihydro-1H-inden-1-yl}oxy)-3-fluorobenzonitrile
4-({(1R,2R)-2-[(3R)-3-aminopiperidin-1-yl]-2,3-dihydro-1H-inden-1-yl}sulfanyl)benzonitrile
4-({(1R,2R)-2-[(3S)-3-aminopiperidin-1-yl]-2,3-dihydro-1H-inden-1-yl}oxy)benzonitrile
4-({(1R,2R)-2-[(3S)-3-aminopiperidin-1-yl]-2,3-dihydro-1H-inden-1-yl}oxy)-2-fluorobenzonitrile
4-({(1R,2R)-2-[(3S)-3-aminopiperidin-1-yl]-2,3-dihydro-1H-inden-1-yl}oxy)-3-fluorobenzonitrile
4-({(1R,2R)-2-[(3S)-3-aminopiperidin-1-yl]-2,3-dihydro-1H-inden-1-yl}oxy)-3-chlorobenzonitrile
4-({(1R,2R)-2-[(3S)-3-aminopiperidin-1-yl]-2,3-dihydro-1H-inden-1-yl}oxy)-2,3-difluorobenzonitrile
4-({(1R,2R)-2-[(3S)-3-aminopiperidin-1-yl]-2,3-dihydro-1H-inden-1-yl}oxy)-3-(trifluoromethyl)benzonitrile
(3S)-1-[(1R,2R)-1-(2-chloro-6-methylphenoxy)-2,3-dihydro-1H-inden-2-yl]piperidin-3-amine
(3S)-1-{(1R,2R)-1-[2-(2H-benzotriazol-2-yl)-4-methylphenoxy]-2,3-dihydro-1H-inden-2-yl}piperidin-3-amine
4-({(1R,2R)-2-[(3S)-3-aminopiperidin-1-yl]-2,3-dihydro-1H-inden-1-yl}oxy)-3-nitrobenzonitrile
2-({(1R,2R)-2-[(3S)-3-aminopiperidin-1-yl]-2,3-dihydro-1H-inden-1-yl}oxy)-5-bromobenzonitrile
2-({(1R,2R)-2-[(3S)-3-aminopiperidin-1-yl]-2,3-dihydro-1H-inden-1-yl]oxy)-5-chlorobenzonitrile
(3S)-1-[(1R,2R)-1-(2-bromo-4,6-difluorophenoxy)-2,3-dihydro-1H-inden-2-yl]piperidin-3-amine
4-({(1R,2R)-2-[(3S)-3-aminopiperidin-1-yl]-2,3-dihydro-1H-inden-1-yl}oxy)-3-bromobenzonitrile
1-[(1R,2R)-1-(2-bromophenoxy)-2,3-dihydro-1H-inden-2-yl]-1,4-diazepane
1-[(1R,2R)-1-(2-chloro-6-methylphenoxy)-2,3-dihydro-1H-inden-2-yl]-1,4-diazepane
1-[(1R,2R)-1-(2-bromo-4-fluorophenoxy)-2,3-dihydro-1H-inden-2-yl]-1,4-diazepane
1-[(1R,2R)-1-(2,3,6-trifluorophenoxy)-2,3-dihydro-1H-inden-2-yl]-1,4-diazepane
1-[(1R,2R)-1-(2,4,6-trifluorophenoxy)-2,3-dihydro-1H-inden-2-yl]-1,4-diazepane
1-[(1R,2R)-1-(2-bromo-4,6-difluorophenoxy)-2,3-dihydro-1H-inden-2-yl]-1,4-diazepane
(3R)-1-[(1R,2R)-1-(2,4-dichlorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)benzonitrile
4-({(1R,2R)-2-[(3R)-3-aminopiperidin-1-yl]-2,3-dihydro-1H-inden-1-yl}oxy)-3-fluorobenzonitrile
4-({(1R,2R)-2-[(3R)-3-aminopiperidin-1-yl]-2,3-dihydro-1H-inden-1-yl}oxy)-2-chlorobenzonitrile
4-({(1R,2R)-2-[(3R)-3-aminopiperidin-1-yl]-2,3-dihydro-1H-inden-1-yl}oxy)-2,3-difluorobenzonitrile
4-({(1R,2R)-2-[(3R)-3-aminopiperidin-1-yl]-2,3-dihydro-1H-inden-1-yl}oxy)-3-(trifluoromethyl)benzonitrile
4-({(1R,2R)-2-[(3R)-3-aminopiperidin-1-yl]-2,3-dihydro-1H-inden-1-yl}oxy)-3-methoxybenzonitrile
(3R)-1-[(1R,2R)-1-(4-chloro-2-methylphenoxy)-2,3-dihydro-1H-inden-2-yl]piperidin-3-amine
(3R)-1-[(1R,2R)-1-(2,4-difluorophenoxy)-2,3-dihydro-1H-inden-2-yl]piperidin-3-amine
(3R)-1-[(1R,2R)-1-(4-bromo-3-fluorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)-3-nitrobenzonitrile
(3R)-1-[(1R,2R)-1-(4-fluoro-2-methylphenoxy)-2,3-dihydro-1H-inden-2-yl]piperidin-3-amine
2-({(1R,2R)-2-[(3R)-3-aminopiperidin-1-yl]-2,3-dihydro-1H-inden-1-yl}oxy)-5-bromobenzonitrile
2-({(1R,2R)-2-[(3R)-3-aminopiperidin-1-yl]-2,3-dihydro-1H-inden-1-yl}oxy)-5-chlorobenzonitrile
5-({(1R,2R)-2-[(3R)-3-aminopiperidin-1-yl]-2,3-dihydro-1H-inden-1-yl}oxy)-1,3-benzoxathiol-2-one
2-({(1R,2R)-2-[(3R)-3-aminopiperidin-1-yl]-2,3-dihydro-1H-inden-1-yl}oxy)-6-fluorobenzonitrile
(3R)-1-{(1R,2R)-1-[4-bromo-2-(1H-pyrazol-3-yl)phenoxy]-2,3-dihydro-1H-inden-2-yl}piperidin-3-amine
4-({(1R,2R)-2-[(3R)-3-aminopiperidin-1-yl]-2,3-dihydro-1H-inden-1-yl}oxy)-3-ethoxybenzonitrile
2-({(1R,2R)-2-[(3R)-3-aminopiperidin-1-yl]-2,3-dihydro-1H-inden-1-yl}oxy)benzonitrile
(3R)-1-[(1R,2R)-1-(4-bromophenoxy)-2,3-dihydro-1H-inden-2-yl]piperidin-3-amine
(3R)-1-[(1R,2R)-1-(4-fluorophenoxy)-2,3-dihydro-1H-inden-2-yl]piperidin-3-amine
(3R)-1-[(1R,2R)-1-(2-bromo-4-chlorophenoxy)-2,3-dihydro-1H-inden-2-yl]piperidin-3-amine
2-({(1R,2R)-2-[(3R)-3-aminopiperidin-1-yl]-2,3-dihydro-1H-inden-1-yl}oxy)-5-chlorobenzamide
6-({(1R,2R)-2-[(3R)-3-aminopiperidin-1-yl]-2,3-dihydro-1H-inden-1-yl}oxy)-3,4-dihydroquinolin-2(1H)-one
(3R)-1-{(1R,2R)-1-[4-methyl-2-(1H-pyrazol-3-yl)phenoxy]-2,3-dihydro-1H-inden-2-yl}piperidin-3-amine
3-({(1R,2R)-2-[(3R)-3-aminopiperidin-1-yl]-2,3-dihydro-1H-inden-1-yl}oxy)-N,N-dimethylnaphthalene-2-carboxamide
2-({(1R,2R)-2-[(3R)-3-aminopiperidin-1-yl]-2,3-dihydro-1H-inden-1-yl}oxy)-4-bromobenzonitrile
2-({(1R,2R)-2-[(3S)-3-aminopiperidin-1-yl]-2,3-dihydro-1H-inden-1-yl}oxy)-6-fluorobenzonitrile
4-({(1R,2R)-2-[(3R)-3-aminopyrrolidin-1-yl]-2,3-dihydro-1H-inden-1-yl}oxy)benzonitrile
4-({(1R,2R)-2-[(3R)-3-aminopyrrolidin-1-yl]-2,3-dihydro-1H-inden-1-yl}oxy)-3-fluorobenzonitrile
4-({(1R,2R)-2-[(3R)-3-aminopyrrolidin-1-yl]-2,3-dihydro-1H-inden-1-yl}oxy)-3-chlorobenzonitrile
(3R)-1-{(1R,2R)-1-[2-(2H-benzotriazol-2-yl)-4-methylphenoxy]-2,3-dihydro-1H-inden-2-yl}pyrrolidin-3-amine
4-({(1R,2R)-2-[(3R)-3-aminopyrrolidin-1-yl]-2,3-dihydro-1H-inden-1-yl}oxy)-3-nitrobenzonitrile
(3R)-1-[(1R,2R)-1-(2-bromo-4-fluorophenoxy)-2,3-dihydro-1H-inden-2-yl]piperidin-3-amine
(3R)-1-{(1R,2R)-1-[4-chloro-2-(1H-pyrazol-3-yl)phenoxy]-2,3-dihydro-1H-inden-2-yl}piperidin-3-amine
(3R)-1-{(1R,2R)-1-[4-chloro-2-(1,2-oxazol-5-yl)phenoxy]-2,3-dihydro-1H-inden-2-yl}piperidin-3-amine
(3R)-1-[(1R,2R)-1-(2-bromo-4,6-difluorophenoxy)-2,3-dihydro-1H-inden-2-yl]piperidin-3-amine
(3R)-1-[(1R,2R)-1-(4-chloro-2-fluorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)-3-bromobenzonitrile
(3R)-1-{(1R,2R)-1-[4-bromo-2-(1,2-oxazol-5-yl)phenoxy]-2,3-dihydro-1H-inden-2-yl}piperidin-3-amine
4-({(1R,2R)-2-[(3S)-3-aminopyrrolidin-1-yl]-2,3-dihydro-1H-inden-1-yl}oxy)benzonitrile
4-({(1R,2R)-2-[(3S)-3-aminopyrrolidin-1-yl]-2,3-dihydro-1H-inden-1-yl}oxy)-2-fluorobenzonitrile
4-({(1R,2R)-2-[(3S)-3-aminopyrrolidin-1-yl]-2,3-dihydro-1H-inden-1-yl}oxy)-3-fluorobenzonitrile
4-({(1R,2R)-2-[(3S)-3-aminopyrrolidin-1-yl]-2,3-dihydro-1H-inden-1-yl}oxy)-3-chlorobenzonitrile
4-({(1R,2R)-2-[(3S)-3-aminopyrrolidin-1-yl]-2,3-dihydro-1H-inden-1-yl}oxy)-2,3-difluorobenzonitrile
1-[(1R,2R)-1-(2-chloro-6-fluoro-3-methylphenoxy)-2,3-dihydro-1H-inden-2-yl]-1,4-diazepane
(3S)-1-{(1R,2R)-1-[2-(2H-benzotriazol-2-yl)-4-methylphenoxy]-2,3-dihydro-1H-inden-2-yl}pyrrolidin-3-amine
4-({(1R,2R)-2-[(3S)-3-aminopyrrolidin-1-yl]-2,3-dihydro-1H-inden-1-yl}oxy)-3-nitrobenzonitrile
2-({-2-[(3S)-3-aminopyrrolidin-1-yl]-2,3-dihydro-1H-inden-1-yl}oxy)-5-bromobenzonitrile
2-({(1R,2R)-2-[(3S)-3-aminopyrrolidin-1-yl]-2,3-dihydro-1H-inden-1-yl}oxy)-5-chlorobenzonitrile
4-({(1S,2S)-2-[(3R)-3-aminopiperidin-1-yl]-4-methyl-2,3-dihydro-1H-inden-1-yl}oxy)-3-chlorobenzonitrile
4-({(1R,2R)-2-[3-(methylamino)pyrrolidin-1-yl]-2,3-dihydro-1H-inden-1-yl}oxy)benzonitrile
3-fluoro-4-({(1R,2R)-2-[3-(methylamino)pyrrolidin-1-yl]-2,3-dihydro-1H-inden-1-yl}oxy)benzonitrile
3-chloro-4-({(1R,2R)-2-[3-(methylamino)pyrrolidin-1-yl]-2,3-dihydro-1H-inden-1-yl}oxy)benzonitrile
1-{(1R,2R)-1-[2-(2H-benzotriazol-2-yl)-4-methylphenoxy]-2,3-dihydro-1H-inden-2-yl}-N-methylpyrrolidin-3-amine
1-{(3R)-1-[(1R,2R)-1-(4-nitrophenoxy)-2,3-dihydro-1H-inden-2-yl]pyrrolidin-3-yl}methanamine
1-{(3R)-1-[(1R,2R)-1-(4-bromophenoxy)-2,3-dihydro-1H-inden-2-yl]pyrrolidin-3-yl}methanamine
1-{(3R)-1-[(1R,2R)-1-(2-chloro-4-fluorophenoxy)-2,3-dihydro-1H-inden-2-yl]pyrrolidin-3-yl}methanamine
1-{(3R)-1-[(1R,2R)-1-(2-chloro-4-nitrophenoxy)-2,3-dihydro-1H-inden-2-yl]pyrrolidin-3-yl}methanamine
2-({(1R,2R)-2-[(3R)-3-(aminomethyl)pyrrolidin-1-yl]-2,3-dihydro-1H-inden-1-yl}oxy)benzonitrile
1-{(3R)-1-[(1R,2R)-1-(2-bromo-4-chlorophenoxy)-2,3-dihydro-1H-inden-2-yl]pyrrolidin-3-yl}methanamine
2-({(1R,2R)-2-[(3R)-3-(aminomethyl)pyrrolidin-1-yl]-2,3-dihydro-1H-inden-1-yl}oxy)-5-chlorobenzonitrile
methyl 4-({(1R,2R)-2-[(3R)-3-(aminomethyl)pyrrolidin-1-yl]-2,3-dihydro-1H-inden-1-yl}oxy)-3-chlorobenzoate
4-({(1R,2R)-2-[(3R)-3-(aminomethyl)pyrrolidin-1-yl]-2,3-dihydro-1H-inden-1-yl}oxy)-3-chlorobenzonitrile
1-{(3R)-1-[(1R,2R)-1-(4-bromo-2-chlorophenoxy)-2,3-dihydro-1H-inden-2-yl]pyrrolidin-3-yl}methanamine
1-[(3R)-1-{(1R,2R)-1-[4-bromo-2-(1H-pyrazol-3-yl)phenoxy]-2,3-dihydro-1H-inden-2-yl}pyrrolidin-3-yl]methanamine
2-({(1R,2R)-2-[(3R)-3-(aminomethyl)pyrrolidin-1-yl]-2,3-dihydro-1H-inden-1-yl}oxy)-5-bromobenzonitrile
4-({(1R,2R)-2-[(3R)-3-(aminomethyl)pyrrolidin-1-yl]-2,3-dihydro-1H-inden-1-yl}oxy)-3-bromobenzonitrile
1-[(3R)-1-{(1R,2R)-1-[4-chloro-2-(1H-pyrazol-3-yl)phenoxy]-2,3-dihydro-1H-inden-2-yl}pyrrolidin-3-yl]methanamine
1-[(3R)-1-{(1R,2R)-1-[4-methyl-2-(1H-pyrazol-3-yl)phenoxy]-2,3-dihydro-1H-inden-2-yl}pyrrolidin-3-yl]methanamine
1-{(3R)-1-[(1R,2R)-1-(2,4-dichlorophenoxy)-2,3-dihydro-1H-inden-2-yl]pyrrolidin-3-yl}methanamine
1-{(3R)-1-[(1R,2R)-1-(4-chlorophenoxy)-2,3-dihydro-1H-inden-2-yl]pyrrolidin-3-yl}methanamine
4-({(1R,2R)-2-[(3R)-3-(aminomethyl)pyrrolidin-1-yl]-2,3-dihydro-1H-inden-1-yl}oxy)-2-fluorobenzonitrile
4-({(1R,2R)-2-[(3R)-3-(aminomethyl)pyrrolidin-1-yl]-2,3-dihydro-1H-inden-1-yl}oxy)-3-methoxybenzonitrile
4-({(1R,2R)-2-[(3R)-3-(aminomethyl)pyrrolidin-1-yl]-2,3-dihydro-1H-inden-1-yl}oxy)-2-chlorobenzonitrile
4-({(1R,2R)-2-[(3R)-3-(aminomethyl)pyrrolidin-1-yl]-2,3-dihydro-1H-inden-1-yl}oxy)-2,6-difluorobenzonitrile
2-({(1R,2R)-2-[(3R)-3-(aminomethyl)pyrrolidin-1-yl]-2,3-dihydro-1H-inden-1-yl}oxy)-6-fluorobenzonitrile
4-({(1R,2R)-2-[(3R)-3-(aminomethyl)pyrrolidin-1-yl]-2,3-dihydro-1H-inden-1-yl}oxy)-3-fluorobenzonitrile
4-({(1R,2R)-2-[(3R)-3-(aminomethyl)pyrrolidin-1-yl]-2,3-dihydro-1H-inden-1-yl}oxy)-2,3-difluorobenzonitrile
4-({(1R,2R)-2-[(3R)-3-(aminomethyl)pyrrolidin-1-yl]-2,3-dihydro-1H-inden-1-yl}oxy)-3-(trifluoromethyl)benzonitrile
1-{(3S)-1-[(1R,2R)-1-(4-nitrophenoxy)-2,3-dihydro-1H-inden-2-yl]pyrrolidin-3-yl}methanamine
1-{(3S)-1-[(1R,2R)-1-(2-chloro-4-nitrophenoxy)-2,3-dihydro-1H-inden-2-yl]pyrrolidin-3-yl}methanamine
2-({(1R,2R)-2-[(3S)-3-(aminomethyl)pyrrolidin-1-yl]-2,3-dihydro-1H-inden-1-yl}oxy)-5-chlorobenzonitrile
4-({(1R,2R)-2-[(3S)-3-(aminomethyl)pyrrolidin-1-yl]-2,3-dihydro-1H-inden-1-yl}oxy)-3-chlorobenzonitrile
2-({(1R,2R)-2-[(3S)-3-(aminomethyl)pyrrolidin-1-yl]-2,3-dihydro-1H-inden-1-yl}oxy)-5-bromobenzonitrile
1-{(3S)-1-[(1R,2R)-1-(4-chlorophenoxy)-2,3-dihydro-1H-inden-2-yl]pyrrolidin-3-yl}methanamine
4-({(1R,2R)-2-[(3S)-3-(aminomethyl)pyrrolidin-1-yl]-2,3-dihydro-1H-inden-1-yl}oxy)-2-fluorobenzonitrile
4-({(1R,2R)-2-[(3S)-3-(aminomethyl)pyrrolidin-1-yl]-2,3-dihydro-1H-inden-1-yl}oxy)-2-chlorobenzonitrile
3-chloro-4-({(1R,2R)-2-[4-(dimethylamino)piperidin-1-yl]-2,3-dihydro-1H-inden-1-yl}oxy)benzonitrile
4-({(1R,2R)-2-[(3R)-3-aminopiperidin-1-yl]-6-methoxy-2,3-dihydro-1H-inden-1-yl}oxy)-3-chlorobenzonitrile
(2R,3R)-2-[(3R)-3-aminopiperidin-1-yl]-3-(2-chloro-4-cyanophenoxy)-2,3-dihydro-1H-indene-5-carbonitrile
(1R,2R)-2-[(3R)-3-aminopiperidin-1-yl]-1-(2-chloro-4-cyanophenoxy)-2,3-dihydro-1H-indene-5-carbonitrile
4-{[(1R,2R)-2-[(3R)-3-aminopiperidin-1-yl]-6-(methylsulfonyl)-2,3-dihydro-1H-inden-1-yl]oxy}-3-chlorobenzonitrile
4-({(1R,2R)-2-[(3R)-3-aminopiperidin-1-yl]-5-fluoro-2,3-dihydro-1H-inden-1-yl}oxy)-3-chlorobenzonitrile
4-({(1R,2R)-2-[3-(2-aminopropan-2-yl)azetidin-1-yl]-2,3-dihydro-1H-inden-1-yl}oxy)-3-chlorobenzonitrile
3-chloro-4-({(1R,2R)-2-[(1S,4S)-2,5-diazabicyclo[2.2.1]hept-2-yl]-2,3-dihydro-1H-inden-1-yl}oxy)benzonitrile
methyl 4-({(1R,2R)-2-[(3R)-3-aminopiperidin-1-yl]-2,3-dihydro-1H-inden-1-yl}oxy)-3-chlorobenzoate
3-chloro-4-({(1R,2R)-2-[(3R)-3-(methylamino)piperidin-1-yl]-2,3-dihydro-1H-inden-1-yl}oxy)benzonitrile
1-{(1R,2R)-1-[2-(2H-benzotriazol-2-yl)-4-methylphenoxy]-2,3-dihydro-1H-inden-2-yl}-N-methylpyrrolidin-3-amine
4-({(1R,2R)-2-[(3R)-3-aminopiperidin-1-yl]-6-hydroxy-2,3-dihydro-1H-inden-1-yl}oxy)-3-chlorobenzonitrile
6-({(1R,2R)-2-[(3R)-3-aminopiperidin-1-yl]-6-methoxy-2,3-dihydro-1H-inden-1-yl}oxy)-3,4-dihydroquinolin-2(1H)-one
6-({(1R,2R)-2-[(3R)-3-aminopiperidin-1-yl]-2,3-dihydro-1H-inden-1-yl}oxy)-7-chloro-3,4-dihydroquinolin-2(1H)-one
4-({(1R,2R)-2-[(3R)-3-aminopiperidin-1-yl]-5-bromo-2,3-dihydro-1H-inden-1-yl}oxy)-3-chlorobenzonitrile
4-({(1R,2R)-2-[(3R)-3-aminopiperidin-1-yl]-4,6-dichloro-2,3-dihydro-1H-inden-1-yl}oxy)-3-chlorobenzonitrile
6-({(1R,2R)-2-[(3R)-3-aminopiperidin-1-yl]-5-chloro-6-methoxy-2,3-dihydro-1H-inden-1-yl}oxy)-3,4-dihydroquinolin-2(1H)-one
6-{[(1R,2R)-2-[(3R)-3-aminopiperidin-1-yl]-6-(trifluoromethyl)-2,3-dihydro-1H-inden-1-yl]oxy}-3,4-dihydroquinolin-2(1H)-one
4-({(5R,6R)-6-[(3R)-3-aminopiperidin-1-yl]-6,7-dihydro-5H-cyclopenta[b]pyridin-5-yl}oxy)-3-chlorobenzonitrile
4-({(6R,7S)-6-[(3R)-3-aminopiperidin-1-yl]-6,7-dihydro-5H-cyclopenta[b]pyridin-7-yl}oxy)-3-chlorobenzonitrile
in the form of a free base or of an addition salt with an acid, as well as in the form of an hydrate or of a solvate.

A protecting group Pg, as mentioned hereafter, corresponds to a group which enables, on the one hand, the protection of a reactive function such as an hydroxy or an amine during a synthesis step and, on then other hand, to recover the intact reactive function at the end of the synthesis step. Examples of protecting groups, as well as methods for protecting and deprotecting various functional groups, are given in « Protective Groups in Organic Synthesis », Green et al., 2nd Edition (John Wiley & Sons, Inc., New York).

A leaving group, as mentioned hereafter, corresponds to a group which may easily be cleaved from a molecule by breaking a heterolytic bond, with departure of electronic pair. This group may then easily be replaced by another functional group during a substitution reaction, for example. Such leaving groups may consist in halogen atoms or activated hydroxy groups, such as mesylate, tosylate, triflate or acetyl groups, etc. Examples of leaving groups, as well as references relating to their preparation, are given in « Advances in Organic Chemistry », J. March, 3rd Edition, Wiley Interscience, p. 310-316.

General processes suitable for preparing compounds of the general formula I are described below. The compounds of the formula I can in this connection be prepared by different chemical processes. The groups and radicals A, B, L, X, R1, R2, R3, R4 and R5 and index p mentioned in the following methods have the abovementioned meaning unless they are explicitly defined otherwise.

### Abbreviations:

- HPLC: high performance liquid chromatography
- LC: liquid chromatography
- Rt: retention time
- THF: tetrahydrofuran
- TFA: trifluoroacetic acid
- DMSO: dimethyl sulfoxide
- DMF: dimethylformamide
- AcN: acetonitrile
- RT: room temperature
- min.: minutes
- h: hour(s)
- ES = ESI: electrospray ionization
- MS: Mass Spectroscopy
- HCl: Hydrochloric acid
- EtOAc: Ethyl acetate
- m: multiplet
- bs: broad singlet
- s: singlet

### Method A:

For example, as shown in scheme A that starting from epoxides of the formula II which initially, after epoxide ring opening with an amine of the formula HNR3R4, afford a corresponding 1-amino 2-ol intermediate of the formula III, which is subsequently subjected to a Mitsunobu reaction with an aryl or heteroaryl compounds B-OH which may be substituted one or more times by R5. Phenols are preferably employed in this reaction. It is also possible alternatively to employ aryl or heteroaryl thiols B-SH or aryl- or heteroarylcarboxylic acids B-CO₂H which may be substituted one or more times by R5 in order to obtain the corresponding -S- or -CO₂H- bridged derivatives. Mitsunobu reactions are, as is known, carried out in the presence of a phosphine, e.g. such as triphenylphosphine and of azodicarboxylic esters such as, for example, diisopropyl azodicarboxylate in inert solvents such as acetonitrile, CH₂Cl₂ or tetrahydrofuran. In the case of 1-amino 2-ols of the formula III, this entails migration of the amine residue NR3R4 into position 2 of the basic structure (J. Org. Chem. 1991, 56, 670-672). 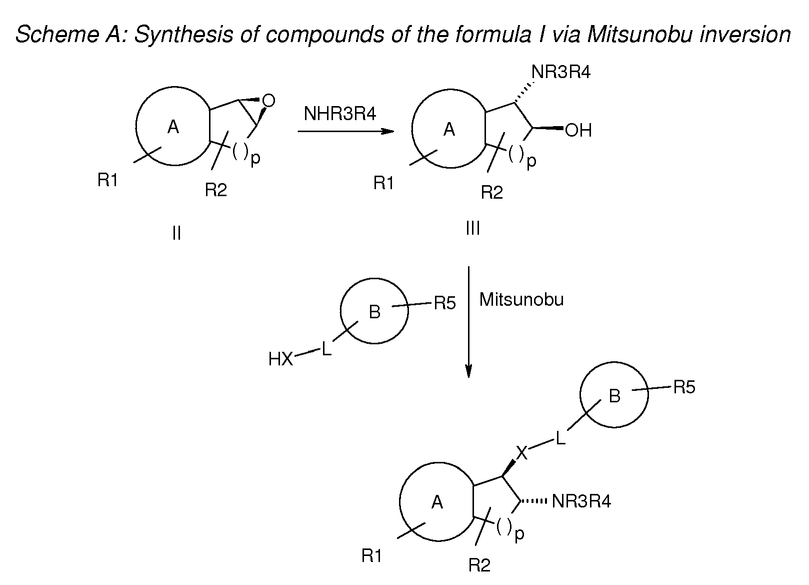 in which L is a covalent bond, -C(=O)- and X is O,
or L is a covalent bond and X is S.

It is possible in this way to prepare a large number of compounds I, preferably those in which the two substituents are in a trans configuration relative to one another. If one of the radicals R3 and R4 of the amine substituent is to be replaced by a further functional group such as, for example, a hydroxy group or an amino group, care must be taken where appropriate to protect such groups during the Mitsunobu reaction. This can take place for example by trialkyl or triarylsilyl groups in the case of OH groups or by the BOC protective groups in the case of amino groups. After the Mitsunobu reaction, the protective group is then removed again, for example by treatment with hydrochloric acid or trifluoroacetic acid, to obtain the compounds of the formula I. After deprotection, these functional groups can be further modified where appropriate, for example by alkylation with an alkylating agent or by acylation and subsequent reduction in order to obtain further compounds I.

The starting materials employed in scheme A, such as the epoxides of the formula II, the amine NHR3R4, and the hydroxyaryls or hydroxyheteroaryls or the thiol derivatives thereof are either commercially available, known from the literature or can be synthesized easily in analogy to compounds known from the literature. A few suitable synthetic schemes for such starting materials are reproduced by way of example in the experimental section.

### Method B:

A further method for preparing compounds of the formula I is depicted in scheme B.

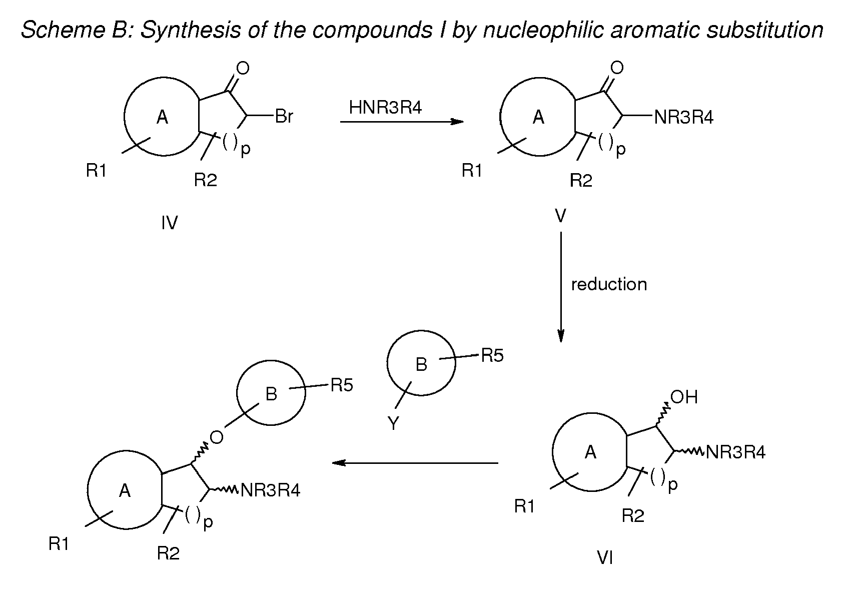

In this process, 2-bromo 1-one compounds of the formula IV are reacted with amines of the formula R3-NH-R4 to give the corresponding amino ketones V. The keto group is then reduced to the 1-hydroxy group, resulting in the intermediates of the formula VI. It is possible in this connection for products VI with both the cis and the trans configuration with regard to centers 1 and 2 to be produced. The resulting intermediates of the formula VI are then arylated by nucleophilic aromatic substitution on aryl or heteroaryl compounds B-Y, where B may be substituted one or more times by R5, using a strong base such as, for example, sodium hydride or powdered NaOH in an inert solvent such as DMSO. Y is in this connection a suitable leaving group such as, for example, fluorine, chlorine or trifluoromesyloxy. If the radicals R3 and R4 are substituted for example by amino or hydroxy groups, these should be protected where appropriate by base-stable protective groups such as, for instance, alkyl- or aryl-substituted silyl groups.

It is also possible with this process to have recourse to a large extent to known or commercially available bromo ketones IV or can easily be obtained for example by bromination under standard conditions from the appropriate ketones.

### Method C:

A further process relates to those compounds of the formula I in which the amine group NR3R4 is linked via a carbon-containing bridge to position 2, that is q is 1 in general formula I.

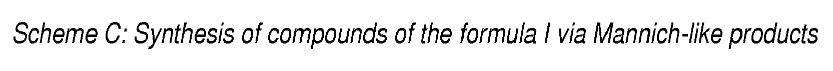

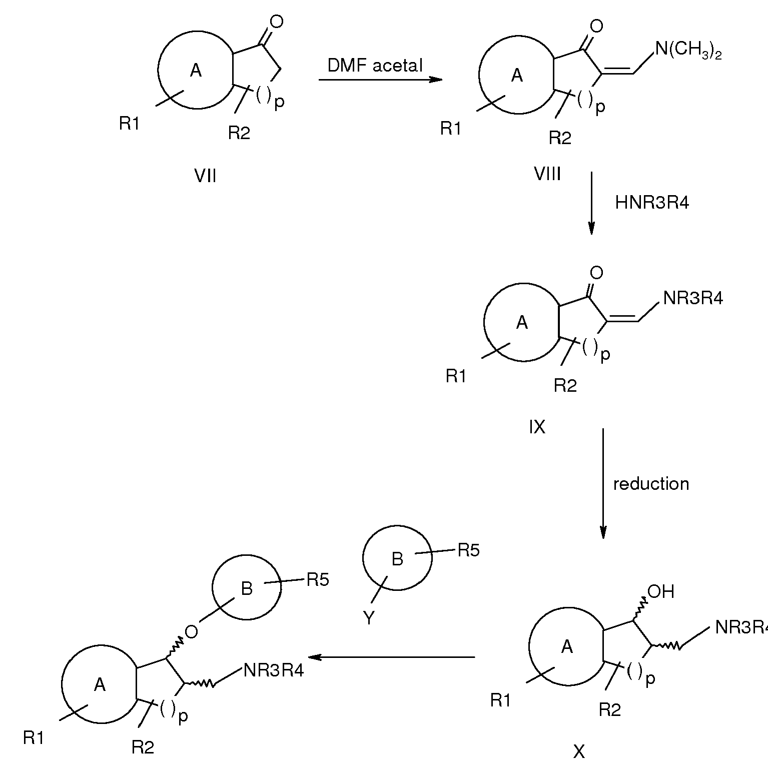

In this case, ketones of the formula VII are reacted with formamide acetals, preferably N,N-dimethylformamide dimethyl acetal, in order to obtain the corresponding dimethylaminomethylene compounds of the formula VIII. The dimethylamino group can be replaced in the next stage by other amino groups to give aminomethylene compounds of the formula IX. This can take place for example by heating compounds of the formula VIII in DMF in the presence of excess amine HNR3R4. Subsequent reduction, for example by sodium borohydride in methanol, ordinarily affords mixtures of stereoisomeric amine alcohols of the formula X which can, where appropriate after separation into the individual components, be arylated in analogy to the illustration in scheme B to give the compounds I of the invention.

### Method D:

A further process for preparing compounds of the formula I is depicted in scheme D. Benzoic esters I which are synthesized as in scheme A are hydrolyzed in a known manner to give compounds of the general formula VI. This takes place for example in solvents such as acetone/water mixtures and using suitable bases such as sodium hydroxide. Compounds of the formula VI are then reacted with suitable alkylating agents such as, for example, benzyl bromides in solvents such as, for example, THF in the presence of suitable bases such as sodium hydride. The compound I obtained in this way is available where appropriate for further manipulations. 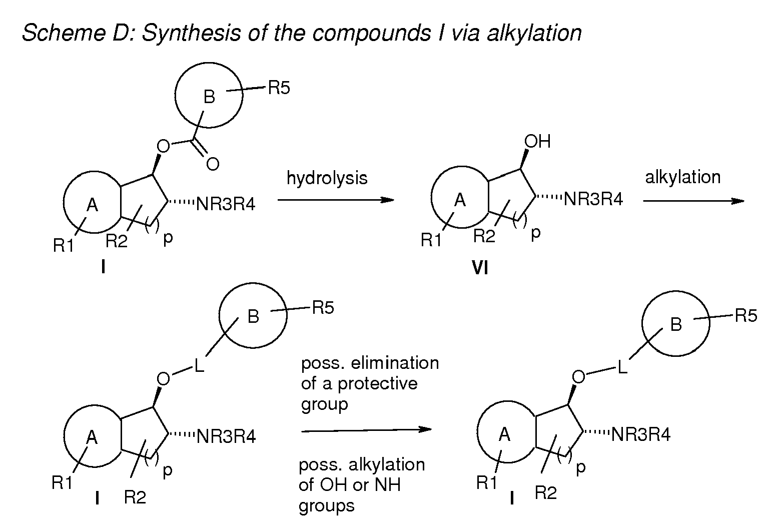 in which L is an alkylene bridge.

If the compounds I contain further functional groups such as, for example, alcohols or amines, these can be reacted further in a known manner as in scheme E. Suitable examples are acylations, alkylations or acylation/reduction sequences. The procedure is described in the experimental section by means of exemplary embodiments.

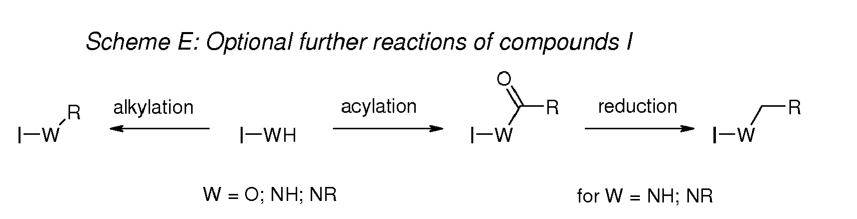

### Method E:

A further process relates to those compounds of the formula I in which one or two substituents R3 or R4 at the amine group NR3R4 equals hydrogen, that is R3 = H or R3 = R4 = H in general formula I.

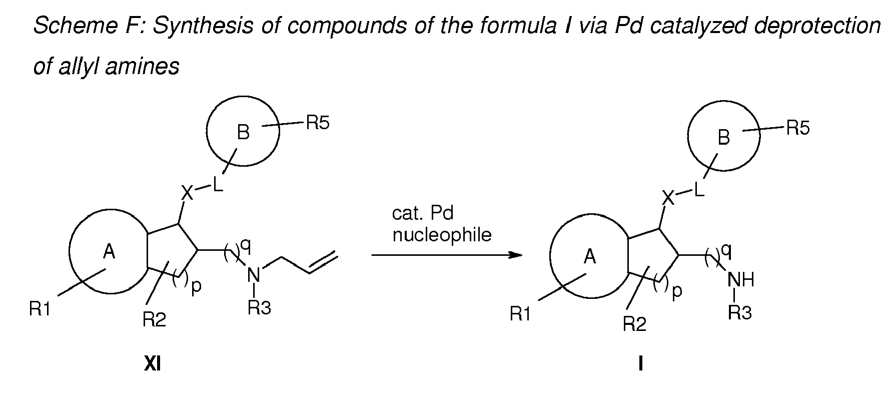

In this process, allyl amines XI, which for example can be synthesized following method A, are deprotected using nucleophiles, e.g. such as thiosalicylic acid or dimethylbarbituric acid, in inert solvents such as CH₂Cl₂ or THF. The reaction is catalyzed by Pd. Suitable Pd sources are for example Pd(PPh₃)₄ or Pd(dba)₂ in the presence of stabilizing ligands such as bis(diphenylphosphino)butane. In case of bisallyl amines (R3 = R4 = allyl) both allyl groups can be cleaved using at least 2 equivalents of a suitable nucleophile and prolonged reaction times. Compounds of the general formula I, which are synthesized following method F, are available for further manipulations e.g. acylation or alkylation.

The following examples describe the synthesis of some compounds according to the invention. These examples are not intended to be limitative and only illustrate the present invention. The numbers of the exemplified compounds refer to those in the table given later, which illustrate the chemical structures and the physical properties of a number of compounds according to the invention.
LC/MS spectra were recorded according to the following methods.

| | |
|---|---|
| Method A: | Solvent: (H₂O+0.05%TFA)/(AcN+0.05%TFA) 98:2 (1min) to 5:95 (5min) to 5:95 (6min) |
| Method B: | Solvent: (H₂O+0,05%TFA)/(AcN+0,035%TFA) 98:2 (1 min) to 0:100 (3min) |
| Method C: | Solvent: (CH₃COONH₄ + 3%AcN)/AcN 100:0 (5 min) to 0:100 (5min) |

### Example 1: (3R)-1-{(1,2-cis)-5-chloro-1-[(2-chloro-5-fluorobenzyl)oxy]-2,3dihydro-1H-inden-2-yl}piperidin-3-amine (compound 1)

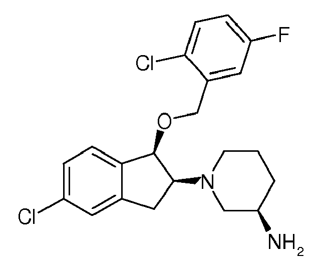

### 1.1 [(R)-1-(cis-5-chloro-1-keto-indan-2-yl)-piperidin-3-yl]-carbamic acid tert-butyl ester

A mixture of 1.00 eq. (20.2 mmol, 4.79 g) 2-bromo-5-chloro-indane-1-one, 1.20 eq. (24.2 mmol, 5.00 g) (R)-3-N-Boc-aminopiperidine and 1.60 eq. (32.0 mmol, 4.47 g) potassium carbonate in 150 ml acetone is stirred at RT for 2 h. After addition of 100 ml of water and 100 ml of EtOAc the layers are separated and the aqueous layer is extracted with EtOAc. The combined organic layers are dried with anhydrous MgSO₄, filtered, concentrated under reduced pressure and the residue is used in the next step without further purification.

### 1.2 [(R)-1-(cis-5-chloro-1-hydroxy-indan-2-yl)-piperidin-3-yl]-carbamic acid tert-butyl ester

[(R)-1-(cis-5-chloro-1-keto-indan-2-yl)-piperidin-3-yl]-carbamic acid tert-butyl ester is dissolved in 150 ml dry THF and 1.50 eq. (30.0 mmol, 30.0 ml of 1 M solution) of L-selectride is added at 0 °C with stirring. The mixture is allowed to warm up to RT and stirring is continued for 2 days. 150 ml water and 150 ml EtOAc are added, the layers are separated and the aqueous layer is extracted with EtOAc. After drying, filtering and concentrating under reduced pressure the combined organic layers, the residue is dissolved in 100 ml THF at 0°C and 5 ml H₂O₂ (35 %) and NaOH (10 ml, 4N) are added. After 2 h at 0°C, water (100 ml) and EtOAc (100 ml) are added. The aqueous layer is extracted with EtOAc. The combined organic layers are dried over anhydrous MgSO₄, filtered and concentrated under reduced pressure. The residue is purified via chromatography on silica gel (EtOAc as eluent) to yield the title compound.

### 1.3 (3R)-1-{(1,2-cis)-5-chloro-1-[(2-chloro-5-fluorobenzyl)oxy]-2,3dihydro-1H-inden-2-yl}piperidin-3-amine

[(R)-1-(cis-5-chloro-1-hydroxy-indan-2-yl)-piperidin-3-yl]-carbamic acid tert-butyl ester (0.1 mmol), 1-chloro-2-(chloromethyl)-4-fluorobenzene (0.2 mmol) and Ag₂O (0.5 mmol) are stirred in 2.5 ml dry toluene for 3h at 85°C. The cooled reaction mixture is filtered and the filtrate is evaporated under reduced pressure. The residue is dissolved in DMF and subjected to preparative HPLC purification.

The purified product is taken up in 1 ml TFA/CH₂Cl₂ (1/9) and shaken for 1 h at RT, then evaporated (12 mbar, 40°C over night in a drying cabinet) to afford the desired product: LCMS (ESI) M⁺ 409.2537.

### Example 2: (3R)-1-{(1R,2R)-1-[2-(2H-benzotriazol-2-yl)-4-methylphenoxy]-2,3-dihydro-1H-inden-2-yl}piperidin-3-amine (compound 7)

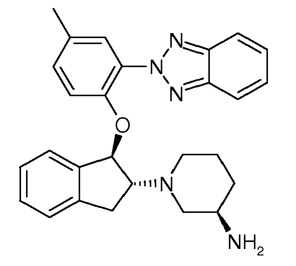

### 2.1 (1aR,6aS)-6,6a-dihydro-1aH-indeno[1,2-b]oxirene

In a three-neck round bottom flask is introduce NaOCl (50.4ml, 0.31 eq., 2N). The flask is then flushed with argon and cooled to 0°C. A solution of (R,R)-N,N'-bis(3,5-di-tert-butylsalicylidene)-1,2-cyclohexanediaminomanganese(III) chloride (2.06g, 0.01eq.) and 4-(3-phenylpropyl)pyridine-N-oxide (2.08g, 0.03eq.) in CH₂Cl₂ (166ml) is added. The suspension is stirred for 15 min. To the cooled solution is added simultaneously *via* two addition funnels NaOCl (152ml, 0.93eq., 2N) and a solution of indene (37.85g, 1eq.) in CH₂Cl₂ (107ml). The mixture is then stirred at 0°C for 1h and the temperature is let warmed up to RT over night. The suspension is diluted with water and CH₂Cl₂ and filtered through celite®. The aqueous layer is separated and extracted three times with CH₂Cl₂. The combined organic layers are washed with brine, dried over anhydrous Na₂SO₄, filtered and concentrated under reduced pressure to afford 18.64g (43%) of the desired compound as a yellow oil, used in the next step without further purification.

### 2.2 tert-butyl {(3R)-1-[(1S,2S)-2-hydroxy-2,3-dihydro-1H-inden-1-yl]piperidin-3-yl}carbamate

To a solution of (1aR,6aS)-6,6a-dihydro-1aH-indeno[1,2-b]oxirene (6g, 1.00eq.) in AcN (122ml) is added tert-butyl (3R)-piperidin-3-ylcarbamate (12.27g, 1.35eq.). The solution is brought to reflux and heated overnight. After cooling, the solvents are evaporated under reduced pressure and the residue is purified by column chromatography (Heptane/EtOAc, 100/0 to 70/30) to afford 12.3g (82%) of the desired product as a solid.

### 2.3 (3R)-1-{(1R,2R)-1-[2-(2H-benzotriazol-2-yl)-4-methylphenoxy]-2,3-dihydro-1H-inden-2-yl}piperidin-3-amine

Tert-butyl {(3R)-1-[(1S,2S)-2-hydroxy-2,3-dihydro-1H-inden-1-yl]piperidin-3-yl}carbamate (3.0 mmol), 2-(2H-benzotriazol-2-yl)-4-methylphenol (3.3 mmol) and triphenylphosphin (5.2 mmol) are dissolved in THF under Argon, then diisopropylazodicarboxylate (4.5 mmol) is added and the mixture is stirred over night at RT. After evaporation of the solvent, the crude product is stirred in 15% TFA in DCM at RT over night. The solvent is evaporated under reduced pressure and the residue is purified by preparative HPLC to afford the desired compound: ¹H NMR (500 MHz, DMSO-d6) δ 8.20 (bs, 2H), 8.05-7.90 (m, 2H), 7.65-7.40 (m, 5H), 7.35-7.15 (m, 4H), 6.25 (s, 1H), 3.90 (s, 1H), 3.40-2.95 (m, 5H), 2.85-2.55 (m, 2H), 2.38 (s, 3H), 2.00-1.83 (m, 2H), 1.70-1.55 (m, 1H), 1.52-1.40 (m, 1H); LCMS (ESI) M⁺ 440.2551.

### Example 3: 4-({(1R,2R)-2-[(3R)-3-aminopiperidin-1-yl]-2,3-dihydro-1H-inden-1-yl}oxy)-3-chlorobenzonitrile (compound 8)

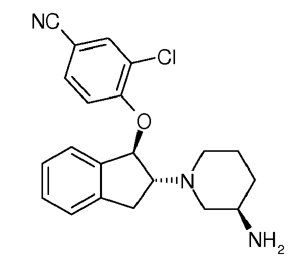

### 3.1 (1aR,6aS)-6,6a-dihydro-1aH-indeno[1,2-b]oxirene

The title compound is prepared following the method used for example 2.

### 3.2 tert-butyl {(3R)-1-[(1S,2S)-2-hydroxy-2,3-dihydro-1H-inden-1-yl]piperidin-3-yl}carbamate

The title compound is prepared following the method used for example 2.

### 3.3 4-({(1R,2R)-2-[(3R)-3-aminopiperidin-1-yl]-2,3-dihydro-1H-inden-1-yl}oxy)-3-chlorobenzonitrile

Tert-butyl {(3R)-1-[(1S,2S)-2-hydroxy-2,3-dihydro-1H-inden-1-yl]piperidin-3-yl}carbamate (0.10 mmol), 3-chloro-4-hydroxybenzonitrile (0.12 mmol) and polymer-bound PPh₃ (Argonaut, 0.25 mmol) in 2.5 ml THF are treated with di-isopropyl azodicarboxylate (0.2 mmol). The mixture is stirred over night at RT then filtered on celite®. The filter is washed with CH₂Cl₂. The filtrate is evaporated under reduced pressure. The residue is purified by column chromatography (CH₂Cl₂/methanol, 100/0 to 90/10) and then dissolved in CH₂Cl₂ and treated with HCl (1ml, 2N in Et2O). The precipitate is filtered and dried under reduced pressure to afford the desired compound as an off-white solid: ¹H NMR (400 MHz, DMSO-d6) δ 8.50 (bs, 2H), 8.18 (s, 1H), 8.05-7.95 (m, 1H), 7.83-7.75 (m, 1H), 7.50-7.40 (m, 2H), 7.32-7.28 (m, 1H), 7.15-7.12 (m, 1H), 6.75 (bs, 1H), 4.18-4.12 (m, 1H), 3.45-3.40 (m, 7H), 1.94-1.90 (m, 4H); LCMS (ES) M⁺ 368; [α_{D}] = -215.5° (CH3OH); mp = 221.3°C.

### Example 4: 4-{[(1R,2R)-2-[(3R)-3-aminopiperidin-1-yl]-6-(methylsulfonyl)-2,3-dihydro-1H-inden-1-yl]oxy}-3-chlorobenzonitrile (compound 123)

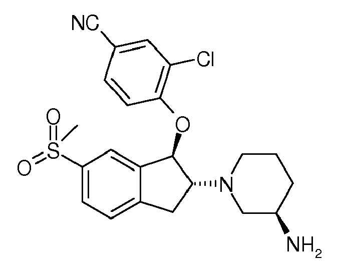

### 4.1 6-(methylsulfonyl)-2,3-dihydro-1H-inden-1-ol

To a cooled solution (0°C) of 6-(methylsulfonyl)-2,3-dihydro-1H-inden-1-one (2.50g, 1eq.) in THF (48ml) and methanol (10ml) is added sodium borohydride (0.89g, 2eq.) portionwise. The suspension is stirred at RT overnight. The mixture is diluted with water. The aqueous layer is extracted with CH₂Cl₂. The organic layer is dried over anhydrous Na₂SO₄, filtered and concentrated under reduced pressure to afford 1.99g (79%) of the desired product as a white solid, used in the next step without further purification.

### 4.2 1H-inden-5-yl methyl sulfone

In a round bottom flask equipped with a dean starck is introduced a solution of 6-(methylsulfonyl)-2,3-dihydro-1H-inden-1-ol (2g, 1 eq.) in toluene (85ml). Para-toluenesulfonic acid (0.02g, 0.01 eq.) is added and the mixture is brought to reflux. The solution is heated for 7h. After cooling, the mixture is diluted with CH₂Cl₂. The organic layer is washed with saturated aqueous NaHCO₃ and water. The organic layer is dried over anhydrous Na₂SO₄, filtered and concentrated under reduced pressure. The residue is purified by column chromatography (CH₂Cl₂/methanol, 100/0 to 95/5) to afford 0.54g (30%) of the desired product as a white solid.

### 4.3 (1aR,6aS)-3-(methylsulfonyl)-6,6a-dihydro-1aH-indeno[1,2-b]oxirene

In a three-neck round bottom flask is introduce NaOCl (0.43ml, 0.31 eq., 2N). The flask is then flushed with argon and cooled to 0°C. A solution of (R,R)-N,N'-bis(3,5-di-tert-butylsalicylidene)-1,2-cyclohexanediaminomanganese(III) chloride (0.02g, 0.01eq.) and 4-(3-phenylpropyl)pyridine-N-oxide (0.02g, 0.03eq.) in CH₂Cl₂ (1ml) is added. The suspension is stirred for 15 min. To the cooled solution is added simultaneously *via* two addition funnels NaOCl (1.3ml, 0.93eq., 2N) and a solution of 1H-inden-5-yl methyl sulfone (0.55g, 1 eq.) in CH₂Cl₂ (0.7ml). The mixture is then stirred at 0°C for 1h and the temperature is let warmed up to RT over night. The suspension is diluted with water and CH₂Cl₂ and filtered through celite®. The aqueous layer is separated and extracted three times with CH₂Cl₂. The combined organic layers are washed with brine, dried over anhydrous Na₂SO₄, filtered and concentrated under reduced pressure to afford 0.51 g (86%) of the desired compound as a brown oil, used in the next step without further purification.

### 4.4 tert-butyl {(3R)-1-[(1S,2S)-2-hydroxy-6-(methylsulfonyl)-2,3-dihydro-1H-inden-1-yl]piperidin-3-yl}carbamate

To a solution of (1aR,6aS)-3-(methylsulfonyl)-6,6a-dihydro-1aH-indeno[1,2-b]oxirene (0.5g, 1.00eq.) in AcN (7ml) is added tert-butyl (3R)-piperidin-3-ylcarbamate (0.69g, 1.45eq.). The solution is brought to reflux and heated overnight. After cooling, the solvents are evaporated under reduced pressure and the residue is purified by column chromatography (CH₂Cl₂/methanol, 100/0 to 95/5) to afford 0.73g (75%) of the desired product as a greenish foam.

### 4.5 4-{[(1R,2R)-2-[(3R)-3-aminopiperidin-1-yl]-6-(methylsulfonyl)-2,3-dihydro-1H-inden-1-yl]oxy}-3-chlorobenzonitrile

To a solution of 3-chloro-4-hydroxybenzonitrile (0.23g, 1.25eq.) in CH₂Cl₂ (7ml) is added polymer-bound PPh₃ (1g, 3mmol/g, 2.50eq.) and tert-butyl {(3R)-1-[(1S,2S)-2-hydroxy-6-(methylsulfonyl)-2,3-dihydro-1H-inden-1-yl]piperidin-3-yl}carbamate (0.50g, 1eq.). The suspension is cooled to 0°C and a solution of di-tert-butyl azodicarboxylate (0.56g, 2eq.) in CH₂Cl₂ (3ml) added dropwise. The mixture is stirred over night at RT, then filtered on celite®. The filter is washed twice with CH₂Cl₂. The filtrate is evaporated under reduced pressure and the residue is purified by column chromatography (CH₂Cl₂/methanol, 100/0 to 95/5) to yield 0.66g (quantitative) of a white foam. The residue is then dissolved in ethanol (1ml) and HCl (0.6ml, 2N in Et₂O) is added. The precipitate is filtered and dried under reduced pressure to afford 0.12g of the desired compound as an off-white solid: ¹H NMR (400 MHz, DMSO-d6) δ 8.38 (bs, 2H), 8.15 (s, 1H), 8.02-8.00 (m, 1H), 8.00-7.98 (m, 1H), 7.88-7.85 (m, 1H), 7.75 (s, 1H), 7.76-7.74 (m, 1H), 6.28 (s, 1H), 3.92-3.88 (m, 1H), 3.35-3.10 (m, 4H), 3.11 (s, 3H), 2.81-2.75 (m, 1H), 2.70-2.51 (m, 2H), 1.95-1.80 (m, 2H), 1.65-1.50 (m, 2H); LCMS (ES) M⁺446; [α_{D}] = -187.1° (CH₃OH); mp = 162 °C.

### Example 5: 4-({(1R,2R)-2-[3-(2-aminopropan-2-yl)azetidin-1-yl]-2,3-dihydro-1H-inden-1-yl}oxy)-3-chlorobenzonitrile (compound 125)

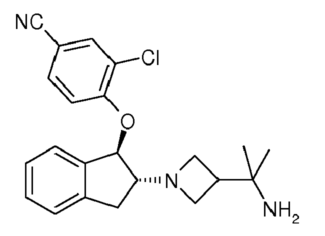

### 5.1 (1aR,6aS)-6,6a-dihydro-1aH-indeno[1,2-b]oxirene

The title compound is prepared following the method used for example 2.

### 5.2 tert-butyl (2-{1-[(1S,2S)-2-hydroxy-2,3-dihydro-1H-inden-1-yl]azetidin-3-yl}propan-2-yl)carbamate

To a solution of (1aR,6aS)-6,6a-dihydro-1aH-indeno[1,2-b]oxirene (0.34g, 1.00eq.) in AcN (7ml) is added tert-butyl [2-(azetidin-3-yl)propan-2-yl]carbamate (0.92g, 1.45eq.). The solution is brought to reflux and heated overnight. After cooling, the solvents are evaporated under reduced pressure and the residue is purified by column chromatography (CH₂Cl₂/methanol, 100/0 to 90/10) to afford 0.47g (53%) of the desired product.

### 5.3 4-({(1R,2R)-2-[3-(2-aminopropan-2-yl)azetidin-1-yl]-2,3-dihydro-1H-inden-1-yl}oxy)-3-chlorobenzonitrile

To a solution of 3-chloro-4-hydroxybenzonitrile (0.26g, 1.25eq.) in CH₂Cl₂ (10ml) is added polymer-bound PPh₃ (1.12g, 3mmol/g, 2.50eq.) and tert-butyl (2-{1-[(1S,2S)-2-hydroxy-2,3-dihydro-1H-inden-1-yl]azetidin-3-yl}propan-2-yl)carbamate (0.47g, 1 eq.). The suspension is cooled to 0°C and a solution of di-isopropyl azodicarboxylate (0.63g, 2eq.) in CH₂Cl₂ (4ml) is added dropwise. The mixture is stirred over night at RT, then filtered on celite®. The filter is washed twice with CH₂Cl₂. The filtrate is evaporated under reduced pressure and the residue is purified by column chromatography (cyclohexane/EtOAc, 100/0 to 70/30) to yield 0.10g (15%) of the product. The residue is then dissolved in ethanol (2 ml) and HCl (1ml, 1 N in Et₂O) is added. The precipitate is filtered and dried under reduced pressure to afford 0.03g of the desired compound as a white solid: ¹H NMR (400 MHz, DMSO-d6) δ 8.25 (bs, 2H), 8.10 (s, 1H), 7.94-7.90 (m, 1H), 7.76-7.74 (m, 1H), 7.46-7.43 (m, 1H), 7.43 (s, 1H), 7.32-7.28 (m, 1H), 7.22-7.19 (m, 1H), 6.45 (s, 1H), 4.80-4.68 (m, 1H), 4.58-4.00 (m, 5H), 1.40-1.25 (m, 2H), 1.20 (s, 6H); LCMS (ES) M⁺ 382; mp = 93°C.

### Example 6: 3-chloro-4-({(1R,2R)-2-[(3R)-3-(methylamino)piperidin-1-yl]-2,3-dihydro-1H-inden-1-yl}oxy)benzonitrile (compound 128)

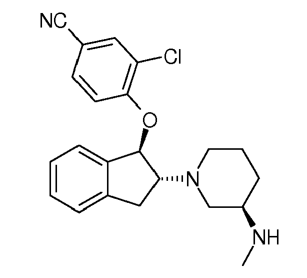

### 6.1 tert-butyl {(3R)-1-[(1R,2R)-1-(2-chloro-4-cyanophenoxy)-2,3-dihydro-1H-inden-2-yl]piperidin-3-yl}carbamate

The title compound is prepared following the method used in example 3.

### 6.2 3-chloro-4-({(1R,2R)-2-[(3R)-3-(methylamino)piperidin-1-yl]-2,3-dihydro-1H-inden-1-yl}oxy)benzonitrile

To a solution of tert-butyl {(3R)-1-[(1R,2R)-1-(2-chloro-4-cyanophenoxy)-2,3-dihydro-1H-inden-2-yl]piperidin-3-yl}carbamate (0.23g, 1eq.) in DMF (1ml) is added water (10µl) and sodium hydride (0.13g, 60% in mineral oil, 7eq.) at O°C. After stirring for 1h at 0°C, methyl iodide (0.09g, 1.50eq.) is added and the mixture is stirred at RT overnight. The mixture is then diluted with CH₂Cl₂ (40ml) and water (5ml). The aqueous layer is separated and extracted with CH₂Cl₂. The combined organic layers are dried over anhydrous Na₂SO₄, filtered and concentrated under reduced pressure. The residue is purified by column chromatography (CH₂Cl₂/EtOAc, 80/20). The product is dissolved in CH₂Cl₂ and treated with HCl (0.10ml, 4N in dioxane). The precipitate is then filtered and dried under vaccum to afford 35mg of the desired product as a beige powder: ¹H NMR (400 MHz, DMSO-d6) δ 9.00 (bs, 1H), 8.00 (s, 1H), 7.90-7.82 (m, 1H), 7.79-7.71 (m, 1H), 7.42-7.40 (m, 1H), 7.40 (s, 1H), 7.30-7.20 (m, 2H), 6.40 (s, 1H), 4.02-3.90 (m, 1H), 3.70-3.20 (m, 4H), 2.92-2.80 (m, 3H), 2.60 (s, 3H), 2.06-1.60 (m, 4H); LCMS (ES) M⁺ 382; [α_{D}] =-197.7° (CH₃OH).

### Example 7: 6-({(1R,2R)-2-[(3R)-3-aminopiperidin-1-yl]-6-methoxy-2,3-dihydro-1H-inden-1-yl}oxy)-3,4-dihydroquinolin-2(1H)-one (compound 131)

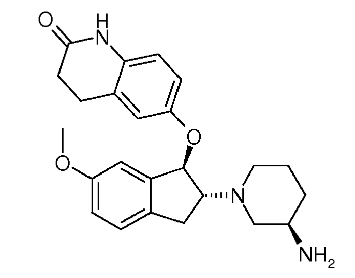

### 7.1 6-methoxy-2,3-dihydro-1H-inden-1-ol

To a cooled solution (0°C) of 6-methoxyindanone (4g, 1eq.) in THF (100ml) and methanol (23ml) is added sodium borohydride (1.86g, 2eq.) portionwise. The suspension is stirred at RT overnight. The mixture is diluted with water. The aqueous layer is extracted with CH₂Cl₂. The organic layer is dried over anhydrous Na₂SO₄, filtered and concentrated under reduced pressure to afford 4.0g (98%) of the desired product as a yellow oil, used in the next step without further purification

### 7.2 1H-inden-5-yl methyl ether

In a round bottom flaky equipped with a dean starck is introduced a solution of 6-methoxy-2,3-dihydro-1H-inden-1-ol (4g, 1eq.) in toluene (121 ml). Para-toluenesulfonic acid (0.05g, 0.01 eq.) is added and the mixture is brought to reflux. The solution is heated for 7h. After cooling, the mixture is diluted with CH₂Cl₂. The organic layer is washed with saturated aqueous NaHCO₃ and water. The organic layer is dried over anhydrous Na₂SO₄, filtered and concentrated under reduced pressure. The residue is purified by column chromatography (heptane/EtOAc, 100/0 to 50/50) to afford 2.69g (75%) of the desired product as acolorless oil.

### 7.3 (1aR,6aS)-3-methoxy-6,6a-dihydro-1aH-indeno[1,2-b]oxirene

In a three-neck round bottom flask is introduce NaOCl (2.8ml, 0.31 eq., 2N). The flask is then flushed with argon and cooled to 0°C. A solution of (R,R)-N,N'-bis(3,5-di-tert-butylsalicylidene)-1,2-cyclohexanediaminomanganese(III) chloride (0.12g, 0.01eq.) and 4-(3-phenylpropyl)pyridine-N-oxide (0.12g, 0.03eq.) in CH₂Cl₂ (9.5ml) is added. The suspension is stirred for 15 min. To the cooled solution is added simultaneously *via* two addition funnels NaOCl (12.5ml, 0.93eq., 2N) and a solution of 1H-inden-5-yl methyl ether (2.69g, 1eq.) in CH₂Cl₂ (5.8ml). The mixture is then stirred at 0°C for 1h and the temperature is let warmed up to RT over night. The suspension is diluted with water and CH₂Cl₂ and filtered through celite®. The aqueous layer is separated and extracted three times with CH₂Cl₂. The combined organic layers are washed with brine, dried over anhydrous Na₂SO₄, filtered and concentrated under reduced pressure to afford 2.90g (97%) of the desired compound as a brown oil, used in the next step without further purification.

### 7.4 tert-butyl {(3R)-1-[(1S,2S)-2-hydroxy-6-methoxy-2,3-dihydro-1H-inden-1-yl]piperidin-3-yl}carbamate

To a solution of (1aR,6aS)-3-methoxy-6,6a-dihydro-1aH-indeno[1,2-b]oxirene (0.70g, 1.00eq) in AcN (16ml) is added tert-butyl (3R)-piperidin-3-ylcarbamate (1.25g, 1.45eq.). The solution is brought to reflux and heated overnight. After cooling, the solvents are evaporated under reduced pressure and the residue is purified by column chromatography (CH₂Cl₂/methanol, 100/0 to 95/5) to afford 0.85g (85%) of the desired product as a brown foam.

### 7.5 6-({(1R,2R)-2-[(3R)-3-aminopiperidin-1-yl]-6-methoxy-2,3-dihydro-1H-inden-1-yl}oxy)-3,4-dihydroquinolin-2(1H)-one

To a solution of 6-hydroxy-3,4-dihydroquinolin-2(1H)-one (0.40g, 1.25eq.) in CH₂Cl₂ (11ml) is added polymer-bound PPh₃ (1.31 g, 3mmol/g, 2.50eq.) and tert-butyl {(3R)-1-[(1S,2S)-2-hydroxy-6-methoxy-2,3-dihydro-1H-inden-1-yl]piperidin-3-yl}carbamate (0.85g, 1eq.). The suspension is cooled to 0°C and a solution of di-tert-butyl azodicarboxylate (0.92g, 2eq.) in CH₂Cl₂ (6.7ml) is added dropwise. The mixture is stirred over night at RT, then filtered on celite®. The filter is washed twice with CH₂Cl₂. The filtrate is evaporated under reduced pressure and the residue is purified by column chromatography (CH₂Cl₂/methanol, 100/0 to 90/10) to yield 0.43g (43%) of an orange foam. The residue is then dissolved in CH₂Cl₂ (5 ml) and HCl (0.82ml, 2N in Et₂O) is added. The precipitate is filtered and dried under reduced pressure to afford 0.25g of the desired compound as an orange solid: ¹H NMR (400 MHz, DMSO-d6) δ 9.55 (s, 1H), 8.30 (bs, 2H), 7.21-7.15 (m, 1H), 6.95 (s, 1H), 6.90-6.75 (m, 3H), 6.60 (s, 1H), 6.05 (s, 1H), 3.94-3.80 (m, 1H), 3.62 (s, 3H), 3.55-2.96 (m, 7H), 2.85-2.75 (m, 2H), 2.40-2.35 (m, 2H), 1.98-1.85 (m, 1H), 1.85-1.70 (m, 2H), 1.65-1.50 (m, 1H); LCMS (ES) M⁺ 408; mp = 145.6 °C.

### Example 8: 6-({(1R,2R)-2-[(3R)-3-aminopiperidin-1-yl]-2,3-dihydro-1H-inden-1-yl}oxy)-7-chloro-3,4-dihydroquinolin-2(1H)-one (compound 132)

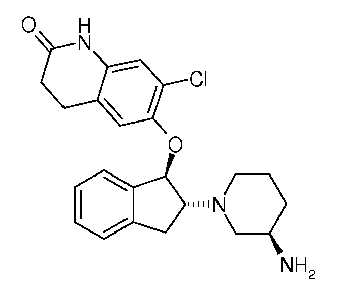

### 8.1 tert-butyl {(3R)-1-[(1S,2S)-2-hydroxy-2,3-dihydro-1H-inden-1-yl]piperidin-3-yl}carbamate

The title compound is prepared following the method used in example 2.

### 8.2 6-({(1R,2R)-2-[(3R)-3-aminopiperidin-1-yl]-2,3-dihydro-1H-inden-1-yl}oxy)-7-chloro-3,4-dihydroquinolin-2(1H)-one

To a solution of 6-hydroxy-7-chloro-3,4-dihydroquinolin-2(1H)-one (0.26g, 1.25eq.) in CH₂Cl₂ (7ml) is added polymer-bound PPh₃ (0.69g, 3mmol/g, 2.50eq.) and tert-butyl {(3R)-1-[(1S,2S)-2-hydroxy-2,3-dihydro-1H-inden-1-yl]piperiden-3-yl}carbamate (0.35g, 1eq.). The suspension is cooled to 0°C and a solution of di-tert-butyl azodicarboxylate (0.48g, 2eq.) in CH₂Cl₂ (3ml) is added dropwise. The mixture is stirred over night at RT, then filtered on celite®. The filter is washed twice with CH₂Cl₂. The filtrate is evaporated under reduced pressure and the residue is purified by column chromatography (CH₂Cl₂/methanol, 100/0 to 92/8) to yield 0.36g (67%) of the product. The residue is then dissolved in CH₂Cl₂ (5 ml) and HCl (0.7ml, 2N in Et₂O) is added. The precipitate is filtered and dried under reduced pressure to afford 0.18g of the desired compound as an orange solid: ¹H NMR (400 MHz, DMSO-d6) δ 9.75 (s, 1H), 8.45 (bs, 2H), 7.42-7.38 (m, 1H), 7.35 (s, 1H), 7.32-7.18 (m, 3H), 7.00 (s, 1H), 6.18 (s, 1H), 4.10-4.00 (m, 1H), 3.60-3.20 (m, 11H), 2.09-2.78 (m, 3H), 1.72-1.60 (m, 1H); LCMS (ES) M⁺ 412; mp = 183.7°C.

### Example 9: 4-({(5R,6R)-6-[(3R)-3-aminopiperidin-1-yl]-6, 7-dihydro-5H-cyclopenta[b]pyridin-5-yl}oxy)-3-chlorobenzonitrile (compound 137)

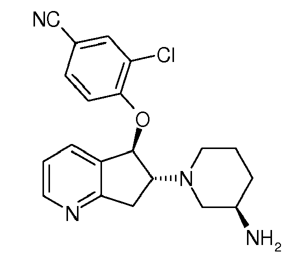

### 9.1 6,7-dihydro-5H-cyclopenta[b]pyridine 1-oxide

To a solution of 6,7-dihydro-5H-cyclopenta[b]pyridine (5g, 1 eq.) in acetic acid (25 ml) is added H₂O₂ (2.51g, 50% in water, 0.88 eq.). The solution is warmed to 70°C. After 3 hours, further H₂O₂ (2.51 g, 50% in water, 0.88 eq.) is added and the mixture is stirred at 70°C over night. After cooling down, the solvent is evaporated under reduced pressure and water (20 ml) is added to the residue. Solid K₂CO₃ is then added to have pH=9. The aqueous layer is separated and extracted three times with CH₂Cl₂. The combined organic layers are dried over anhydrous Na₂SO₄, filtered and evaporated under reduced pressure to yield 5.55g (97%) of the desired compound, used in the next step without further purification.

### 9.2 6,7-dihydro-5H-cyclopenta[b]pyridin-7-yl acetate

To a solution of acetic anhydride (44g, 10.50eq.) and water (0.42g, 0.50eq.) is added 6,7-dihydro-5H-cyclopenta[b]pyridine 1-oxide (5.55g, 1 eq.). After stirring for 1h at RT, the mixture is heated gently to 80°C. The temperature is monitored in order not to rise above 95°C. When the exothermy is over, the Brownish red solution is heated to 100°C for 3h. After cooling to RT, water (150ml) and Et₂O (300ml) are added. The aqueous layer is separated and extracted three times with Et₂O. The combined organic layers are dried over Na₂SO₄, filtered and evaporated under reduced pressure to yield 3.75g (87%) of the desired compound, used in the next step without further purification.

### 9.3 5H-cyclopenta[b]pyridine and 7H-cyclopenta[b]pyridine

Concentrated sulphuric acid (20.5ml, 6.63eq.) is added to 6,7-dihydro-5H-cyclopenta[b]pyridin-7-yl acetate (5.3g, 1eq.). The mixture is stirred at 130°C for 1h then stirred at RT overnight. Ice is added, followed by sodium hydroxide (25ml, 35%) and water (120ml). The aqueous layer is extracted 3 times with CH₂Cl₂. The combined organic layers are dried over anhydrous Na₂SO₄, filtered and concentrated under reduced pressure to obtain 3.2g (95%) of a 65:35 mixture of the 2 regioisomers as a black oil, used in the next step without further purification.

### 9.4 (1aR,6aS)-6,6a-dihydro-1aH-oxireno[4,5]cyclopenta[1,2-b]pyridine and (1 aS,6aR) 2,6b-dihydro-1aH-oxireno[3,4]cyclopenta[1,2-b]pyridine

In a three-neck round bottom flask is introduce NaOCl (7.1 ml, 1 eq., 2N). The flask is then flushed with argon and cooled to 0°C. A solution of (R,R)-N,N'-bis(3,5-di-tert-butylsalicylidene)-1,2-cyclohexanediaminomanganese(III) chloride (0.17g, 0.01eq.) and 4-(3-phenylpropyl)pyridine-N-oxide (0.17g, 0.03eq.) in CH₂Cl₂ (8ml) is added. The suspension is stirred for 15 min. Simultaneous addition of NaOCl (10ml, 1.2eq., 2N) and a solution of 5H-cyclopenta[b]pyridine and 7H-cyclopenta[b]pyridine (3.3g, 1eq.) in CH₂Cl₂ (8ml) *via* two addition funnel follows. The mixture is then stirred at 0°C for 1h and the temperature is let warmed up to RT over night. The suspension is diluted with water and CH₂Cl₂ and filtered through celite®. The aqueous layer is separated and extracted three times with CH₂Cl₂. The combined organic layers are washed with brine, dried over anhydrous Na₂SO₄, filtered and concentrated under reduced pressure to afford 3.70g (quantitative) of a 65:35 mixture of the 2 desired regioisomers, used in the next step without further purification.

### 9.5 tert-butyl {(3R)-1-[(5S,6S)-6-hydroxy-6,7-dihydro-5H-cyclopenta[b]pyridin-5-yl]piperidin-3-yl}carbamate and tert-butyl {(3R)-1-[(6S,7S)-6-hydroxy-6,7-dihydro-5H-cyclopenta[b]pyridin-7-yl]piperidin-3-yl}carbamate

To a solution of (1aR,6aS)-6,6a-dihydro-1aH-oxireno[4,5]cyclopenta[1,2-b]pyridine and (1aS,6aR)-2,6b-dihydro-1aH-oxireno[3,4]cyclopenta[1,2-b]pyridine (3.70g, 1eq.) in AcN (70ml) is added tert-butyl (3R)-piperidine-3-yl carbamate (7.98g, 1.43eq.). The solution is brought to reflux and heated over night. After cooling, the mixture is evaporated under reduced pressure and the resulting residue is purified by column chromatography (CH₂Cl₂/methanol, 100/0 to 90/10) to afford 3.08g of tert-butyl {(3R)-1-[(5S,6S)-6-hydroxy-6,7-dihydro-5H-cyclopenta[b]pyridin-5-yl]piperidin-3-yl}carbamate and 1.85g of tert-butyl {(3R)-1-[(6S,7S)-6-hydroxy-6,7-dihydro-5H-cyclopenta[b]pyridin-7-yl]piperidin-3-yl}carbamate.

### 9.6 4-({(5R,6R)-6-[(3R)-3-aminopiperidin-1-yl]-6,7-dihydro-5H-cyclopenta[b]pyridin-5-yl}oxy)-3-chlorobenzonitrile

To a solution of 3-chloro-4-hydroxybenzonitrile (0.48g, 1.05eq.) in THF (36ml) is added polymer-bound PPh₃ (2.36g, 3mmol/g, 3eq.) and tert-butyl {(3R)-1-[(5S,6S)-6-hydroxy-6,7-dihydro-5H-cyclopenta[b]pyridin-5-yl]piperidin-3-yl}carbamate (1g, 1eq.). The suspension is cooled to 0°C and a solution of di-isopropyl azodicarboxylate (1.82g, 3eq.) in THF (3ml) is added dropwise. The mixture is stirred over night at RT, then filtered on celite®. The filter is washed twice with CH₂Cl₂. The filtrate is evaporated under reduced pressure and the residue is purified by column chromatography (CH₂Cl₂/methanol, 100/0 to 95/5) to yield 0.2g of the desired product. The residue is then dissolved in CH₂Cl₂ (1.4ml) and HCl (0.5ml, 4N in Et₂O) is added. The precipitate is filtered and dried under reduced pressure to afford 0.10g of the desired compound as light grey solid: ¹H NMR (400 MHz, DMSO-d6) δ 8.60-8.55 (m, 1H), 8.32 (bs, 2H), 8.02 (s, 1H), 7.92-7.88 (m, 1H), 7.83-7.79 (m, 1H), 7.65-7.60 (m, 1H), 7.37-7.28 (m, 1H), 6.56 (s, 1H), 4.25-4.15 (m, 1H), 3.58-3.35 (m, 4H), 3.12-3.00 (m, 1H), 3.00-2.80 (m, 2H), 2.06-1.60 (m, 4H); LCMS (ES) M⁺ 369; [α_{D}] = -113 ° (CH₃OH); mp = 172°C.

### Example 10: 4-({(6R,7S)-6-[(3R)-3-aminopiperidin-1-yl]-6,7-dihydro-5H-cyclopenta[b]pyridin-7-yl}oxy)-3-chlorobenzonitrile (compound 138)

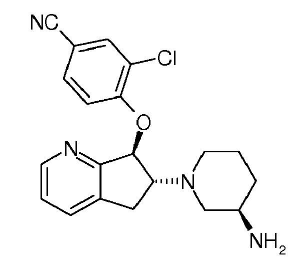

To a solution of 3-chloro-4-hydroxybenzonitrile (0.72g, 1.05eq.) in THF (53ml) is added polymer-bound PPh₃ (3.54g, 3mmol/g, 3eq.) and tert-butyl {(3R)-1-[(6S,7S)-6-hydroxy-6,7-dihydro-5H-cyclopenta[b]pyridin-7-yl]piperidin-3-yl}carbamate (1.50g, 1eq.). The suspension is cooled to 0°C and a solution of di-isopropyl azodicarboxylate (2.73g, 3eq.) in THF (3ml) is added dropwise. The mixture is stirred over night at RT, then filtered on celite®. The filter is washed twice with CH₂Cl₂. The filtrate is evaporated under reduced pressure and the residue is purified by column chromatography (CH₂Cl₂/methanol, 100/0 to 95/5) to yield 0.5g of the desired product. The residue is then dissolved in CH₂Cl₂ (3.9ml) and HCl (1.5ml, 4N in Et₂O) is added. The precipitate is filtered and dried under reduced pressure to afford 0.18g of the desired product as a white solid: ¹H NMR (400 MHz, DMSO-d6) δ 8.45-8.40 (m, 1H), 8.35 (bs, 2H), 7.99 (s, 1H), 7.90-7.80 (m, 3H), 7.42-7.38 (m, 1H), 6.45 (s, 1H), 4.25-4.15 (m, 1H), 3.58-3.35 (m, 2H), 3.35-3.20 (m, 2H) 3.15-2.80 (m, 3H), 2.06-1.60 (m, 4H); LCMS (ES) M⁺ 369; [α_{D}] = - 245.9° (CH₃OH); mp = 180°C.

The following table illustrates the chemical structures and the physical properties of some examples of compounds according to the present invention. In this table, in the « salt » column, « - » represents a compound as a free base, whereas "TFA" represents a compound in the form of a trifluoroacetic acid salt, « HCl » represents a compound in the form of a hydrochloride, the ratio in parentheses being the acid to base ratio.

| ***N°*** | ***STRUCTURE*** | ***Salt*** | ***Characterization :*** **LCMS retention time minutes (method)** |
|---|---|---|---|
| 1 | 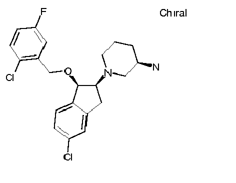 | TFA | 1.25 (A) |
| 2 | 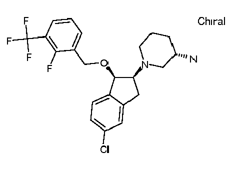 | TFA | 1.34 (A) |
| 3 | 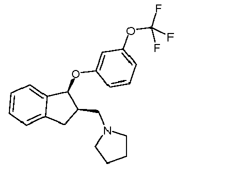 | TFA | 1.78 (A) |
| 4 | 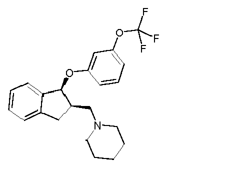 | TFA | 1.80 (A) |
| 5 | 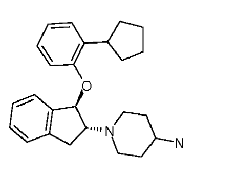 | TFA | 1.32 (A) |
| 6 | 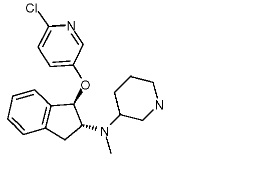 | TFA | 1.06 (A) |
| 7 | 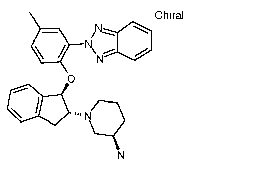 | TFA | 1.29 (A) |
| 8 | 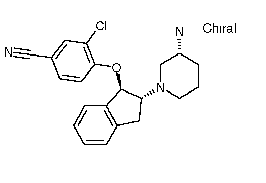 | HCl (2) | 0.85 (B) |
| 9 | 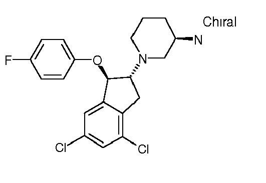 | TFA | 2.72 (A) |
| 10 | 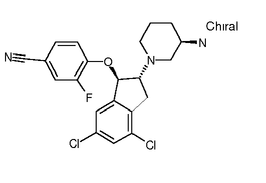 | TFA | 2.80 (A) |
| 11 | 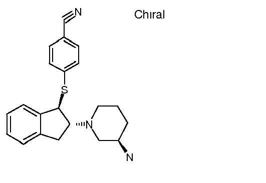 | HCl (2) | 2.40 (A) |
| 12 | 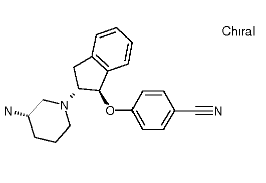 | TFA | 2.27 (A) |
| 13 | 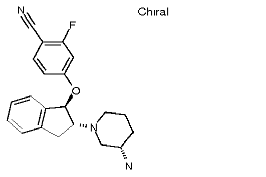 | TFA | 2.37 (A) |
| 14 | 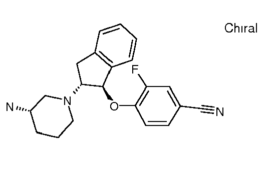 | TFA | 2.34 (A) |
| 15 | 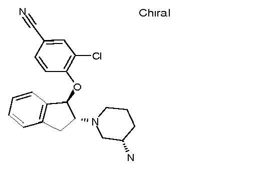 | TFA | 2.42 (A) |
| 16 | 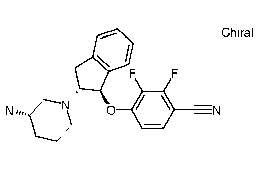 | TFA | 2.47 (A) |
| 17 | 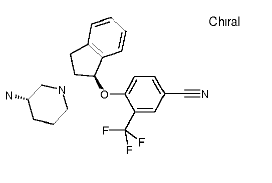 | TFA | 2.52 (A) |
| 18 | 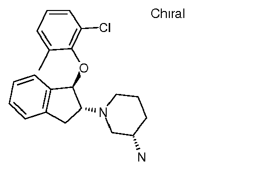 | TFA | 2.43 (A) |
| 19 | 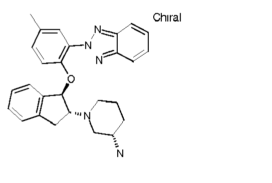 | TFA | 2.60 (A) |
| 20 | 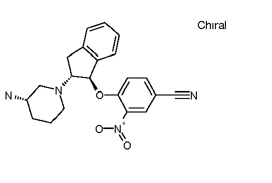 | TFA | 2.37 (A) |
| 21 | 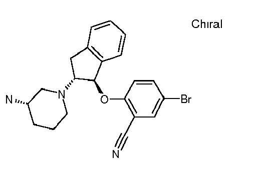 | TFA | 2.50 (A) |
| 22 | 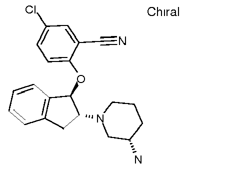 | TFA | 2.47 (A) |
| 23 | 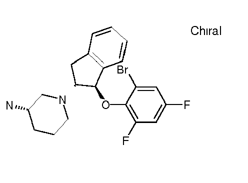 | TFA | 2.48 (A) |
| 24 | 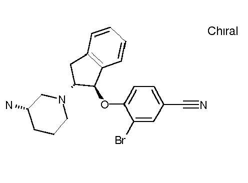 | TFA | 2.43 (A) |
| 25 | 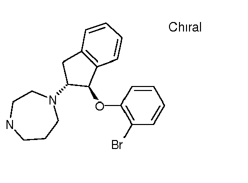 | TFA | 2.45 (A) |
| 26 | 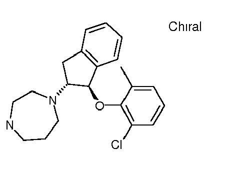 | TFA | 2.48 (A) |
| 27 | 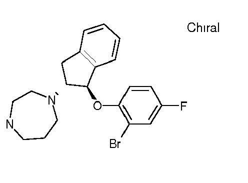 | TFA | 2.54 (A) |
| 28 | 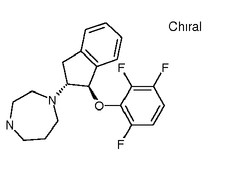 | TFA | 2.45 (A) |
| 29 | 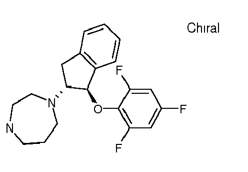 | TFA | 2.40 (A) |
| 30 | 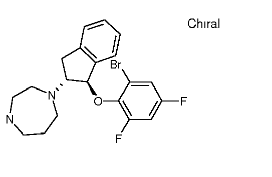 | TFA | 2.54 (A) |
| 31 | 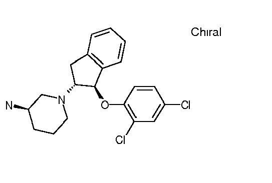 | TFA | 2.60 (A) |
| 32 | 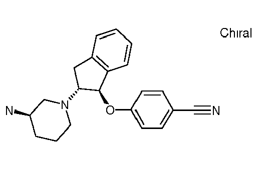 | TFA | 2.29 (A) |
| 33 | 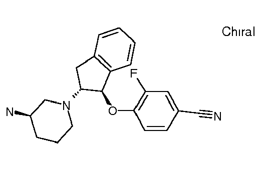 | TFA | 2.40 (A) |
| 34 | 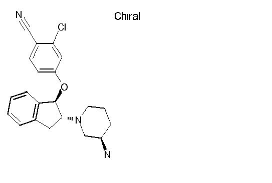 | TFA | 2.47 (A) |
| 35 | 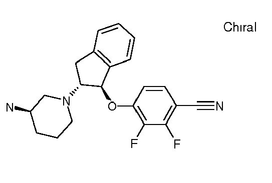 | TFA | 2.48 (A) |
| 36 | 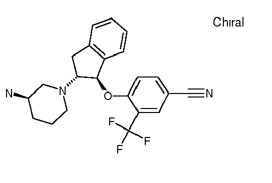 | TFA | 2.52 (A) |
| 37 | 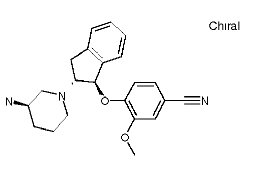 | TFA | 2.27 (A) |
| 38 | 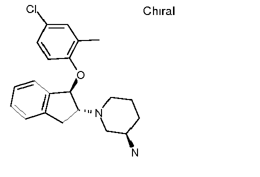 | TFA | 2.60 (A) |
| 39 | 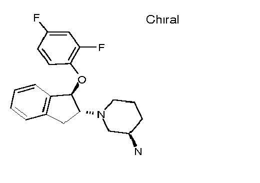 | TFA | 2.37 (A) |
| 40 | 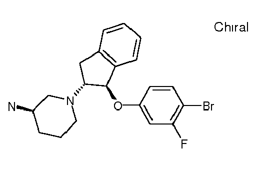 | TFA | 2.55 (A) |
| 41 | 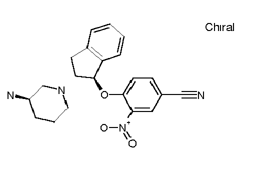 | TFA | 2.32 (A) |
| 42 | 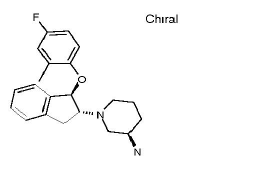 | TFA | 2.48 (A) |
| 43 | 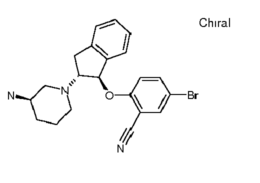 | TFA | 2.52 (A) |
| 44 | 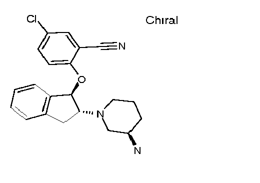 | TFA | 2.47 (A) |
| 45 | 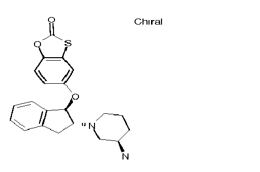 | TFA | 2.43 (A) |
| 46 | 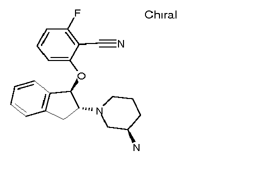 | TFA | 2.42 (A) |
| 47 | 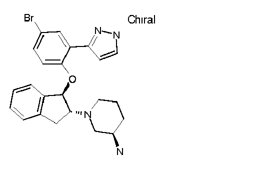 | TFA | 2.50 (A) |
| 48 | 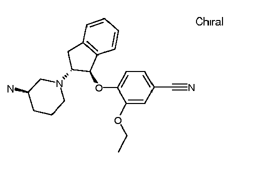 | TFA | 2.40 (A) |
| 49 | 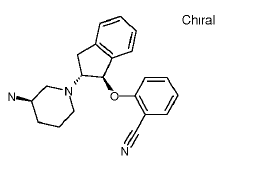 | TFA | 2.29 (A) |
| 50 | 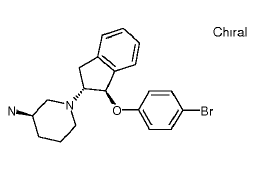 | TFA | 2.50 (A) |
| 51 | 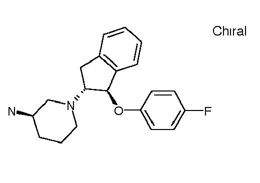 | TFA | 2.34 (A) |
| 52 | 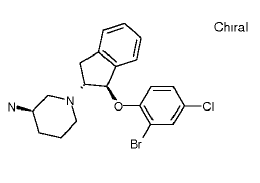 | TFA | 2.62 (A) |
| 53 | 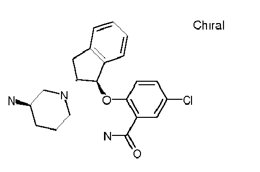 | TFA | 2.25 (A) |
| 54 | 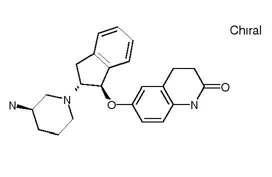 | HCl (2) | 0.68 (B) |
| 55 | 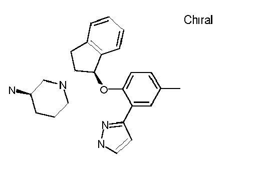 | TFA | 2.69 (A) |
| 56 | 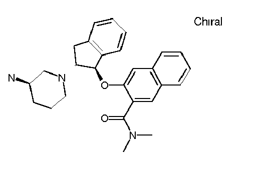 | TFA | 2.48 (A) |
| 57 | 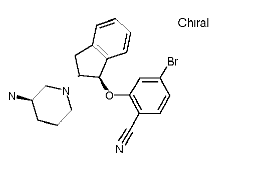 | TFA | 2.48 (A) |
| 58 | 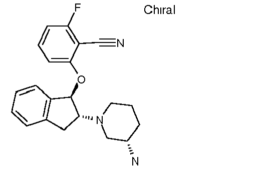 | TFA | 2.32 (A) |
| 59 | 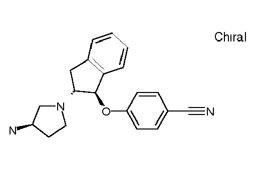 | TFA | 2.30 (A) |
| 60 | 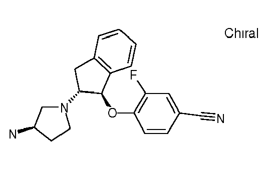 | TFA | 2.29 (A) |
| 61 | 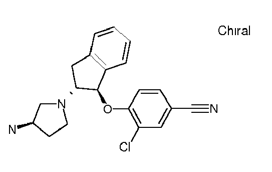 | TFA | 2.40 (A) |
| 62 | 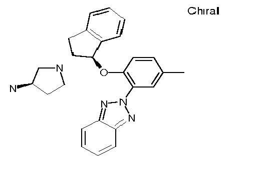 | TFA | 2.60 (A) |
| 63 | 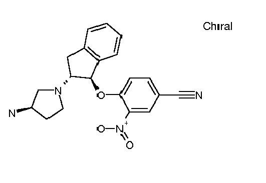 | TFA | 2.30 (A) |
| 64 | 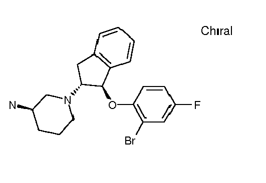 | TFA | 2.52 (A) |
| 65 | 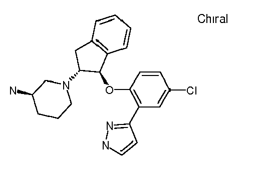 | TFA | 2.47 (A) |
| 66 | 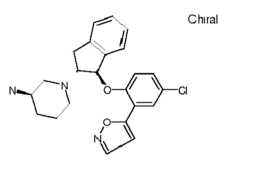 | TFA | 2.62 (A) |
| 67 | 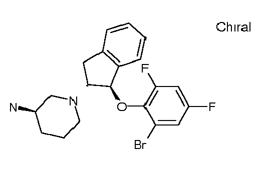 | TFA | 2.43 (A) |
| 68 | 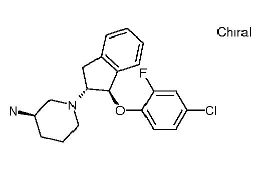 | TFA | 2.52 (A) |
| 69 | 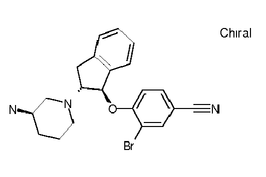 | TFA | 2.45 (A) |
| 70 | 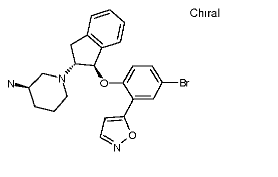 | TFA | 2.62 (A) |
| 71 | 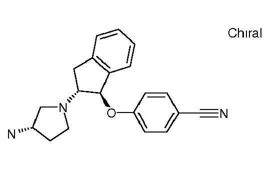 | TFA | 2.27 (A) |
| 72 | 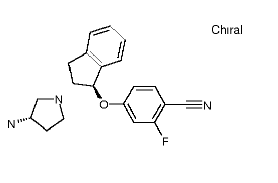 | TFA | 2.34 (A) |
| 73 | 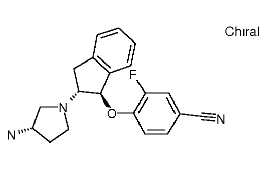 | TFA | 2.30 (A) |
| 74 | 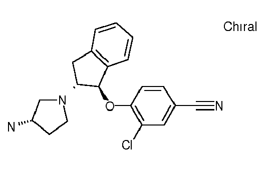 | TFA | 2.34 (A) |
| 75 | 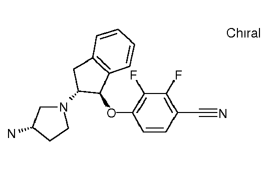 | TFA | 2.42 (A) |
| 76 | 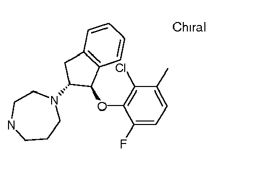 | TFA | 3.32 (A) |
| 77 | 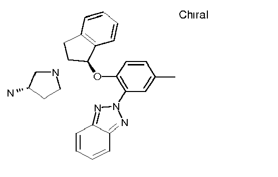 | TFA | 2.55 (A) |
| 78 | 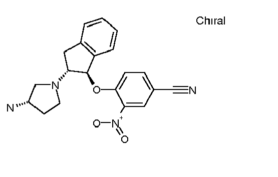 | TFA | 2.32 (A) |
| 79 | 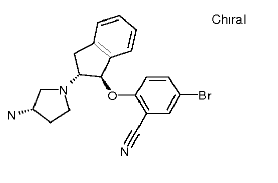 | TFA | 2.50 (A) |
| 80 | 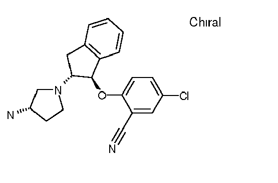 | TFA | 2.40 (A) |
| 81 | 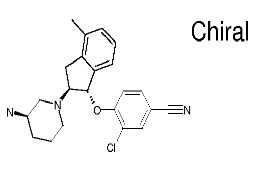 | TFA | 2.52 (A) |
| 82 | 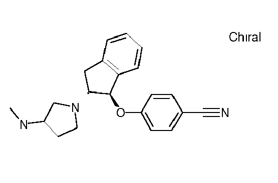 | TFA | 2.30 (A) |
| 83 | 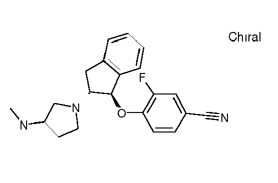 | TFA | 2.37 (A) |
| 84 | 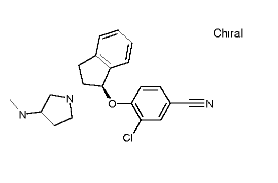 | TFA | 2.45 (A) |
| 85 | 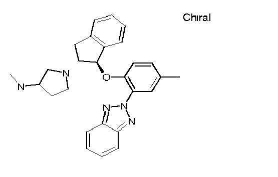 | TFA | 1.91 (A) |
| 86 | 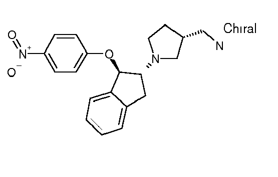 | TFA | 2.34 (A) |
| 87 | 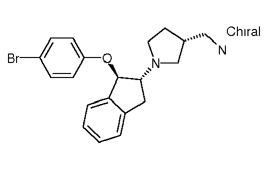 | TFA | 2.50 (A) |
| 88 | 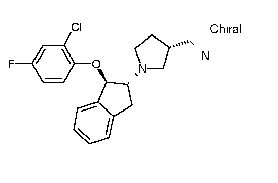 | TFA | 2.40 (A) |
| 89 | 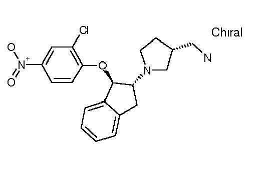 | TFA | 2.43 (A) |
| 90 | 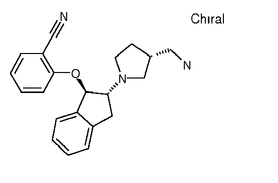 | TFA | 2.25 (A) |
| 91 | 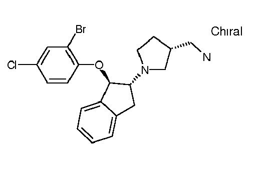 | TFA | 2.59 (A) |
| 92 | 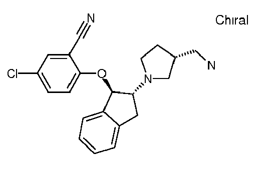 | TFA | 2.45 (A) |
| 93 | 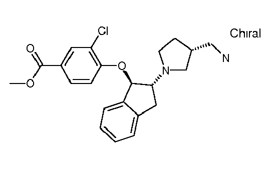 | TFA | 2.43 (A) |
| 94 | 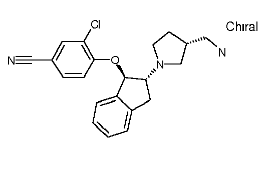 | TFA | 2.35 (A) |
| 95 | 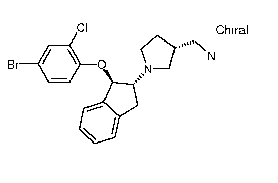 | TFA | 2.59 (A) |
| 96 | 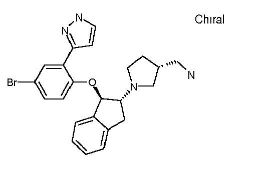 | TFA | 2.48 (A) |
| 97 | 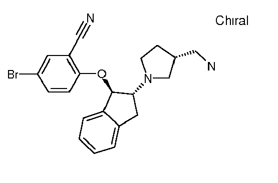 | TFA | 2.47 (A) |
| 98 | 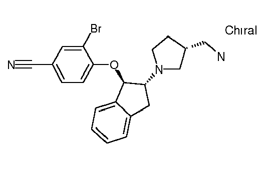 | TFA | 2.42 (A) |
| 99 | 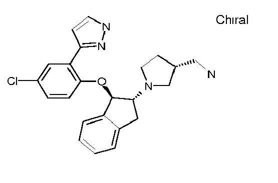 | TFA | 2.43 (A) |
| 100 | 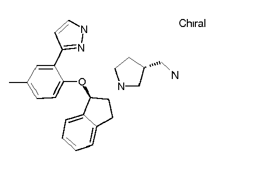 | TFA | 2.40 (A) |
| 101 | 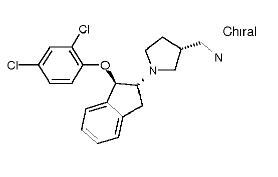 | TFA | 2.54 (A) |
| 102 | 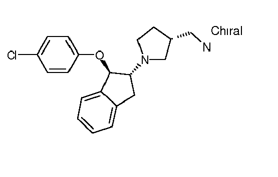 | TFA | 2.48 (A) |
| 103 | 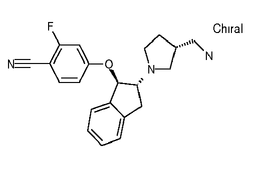 | TFA | 2.32 (A) |
| 104 | 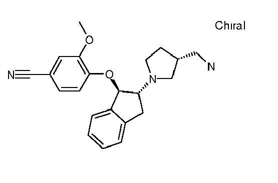 | TFA | 2.29 (A) |
| 105 | 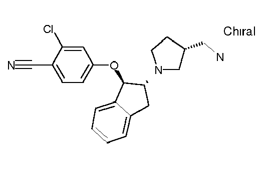 | TFA | 2.40 (A) |
| 106 | 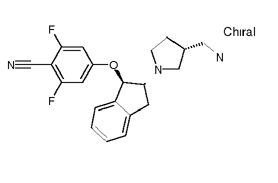 | TFA | 2.42 (A) |
| 107 | 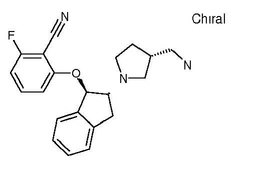 | TFA | 2.37 (A) |
| 108 | 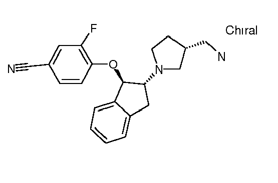 | TFA | 2.27 (A) |
| 109 | 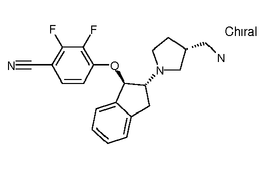 | TFA | 2.39 (A) |
| 110 | 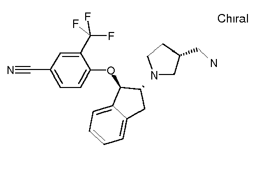 | TFA | 2.45 (A) |
| 111 | 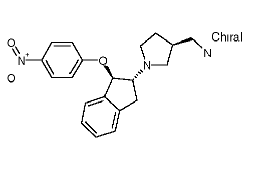 | TFA | 2.32 (A) |
| 112 | 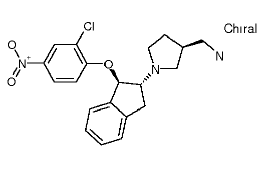 | TFA | 2.43 (A) |
| 113 | 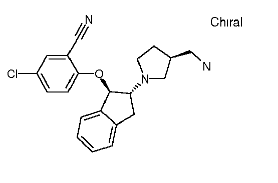 | TFA | 2.43 (A) |
| 114 | 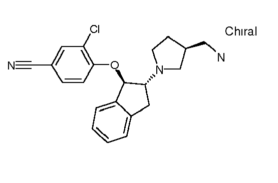 | TFA | 2.18 (A) |
| 115 | 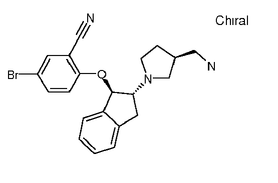 | TFA | 2.47 (A) |
| 116 | 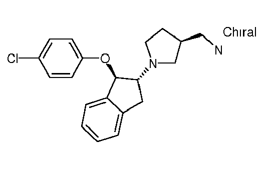 | TFA | 2.42 (A) |
| 117 | 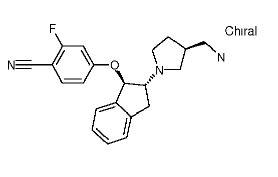 | TFA | 2.30 (A) |
| 118 | 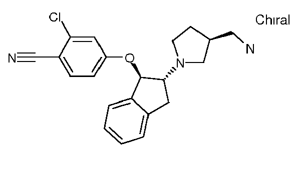 | TFA | 2.39 (A) |
| 119 | 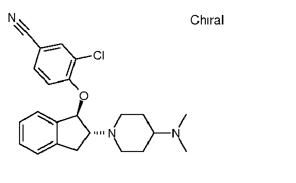 | HCl (2) | 0.74 (B) |
| 120 | 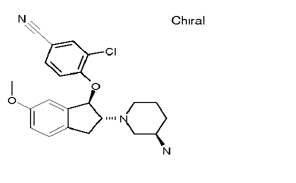 | HCl (2) | 0.79 (B) |
| 121 | 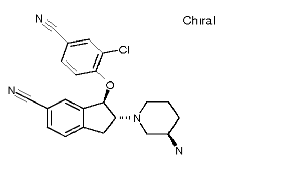 | HCl (2) | 0.80 (B) |
| 122 | 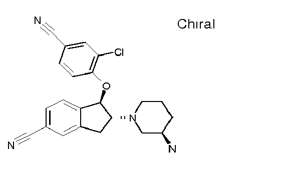 | HCl (2) | 0.94 (B) |
| 123 | 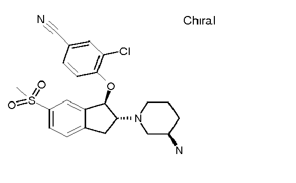 | HCl (2) | 0.79 (B) |
| 124 | 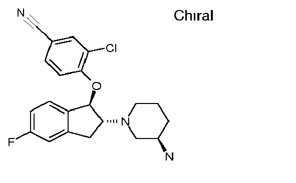 | HCl (2) | 0.90 (B) |
| 125 | 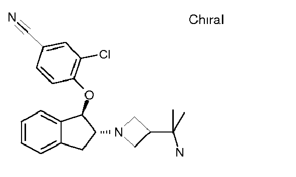 | HCl (2) | 0.82 (B) |
| 126 | 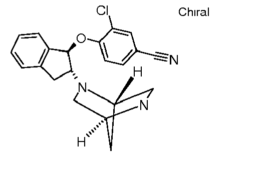 | HCl (2) | 0.88 (B) |
| 127 | 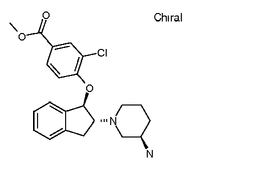 | HCl (2) | 0.88 (B) |
| 128 | 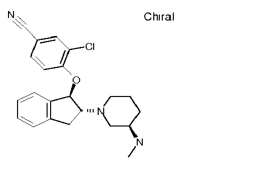 | HCl (2) | 0.90 (B) |
| 129 | 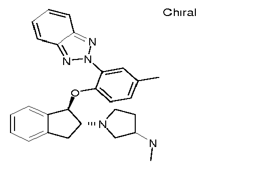 | HCl (2) | 0.96 (B) |
| 130 | 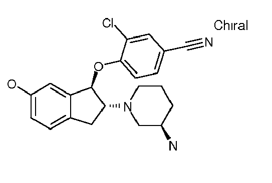 | HCl (2) | 0.77 (B) |
| 131 | 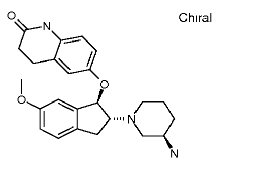 | HCl (2) | 0.66 (B) |
| 132 | 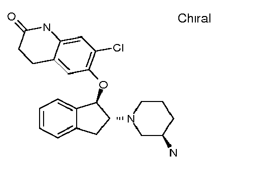 | HCl (2) | 3.17 (C) |
| 133 | 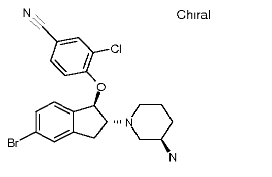 | HCl (2) | 0.93 (B) |
| 134 | 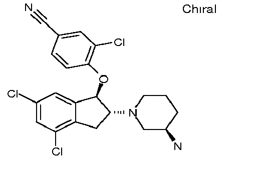 | HCl (2) | 1.12 (B) |
| 135 | 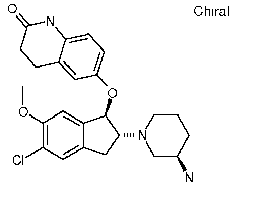 | HCl (2) | 0.78 (B) |
| 136 | 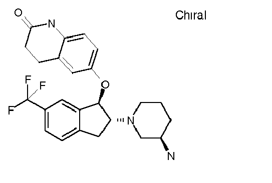 | HCl (2) | 0.82 (B) |
| 137 | 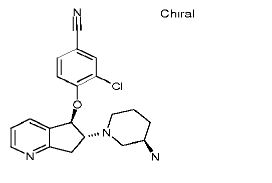 | HCl (2) | 0.66 (B) |
| 138 | 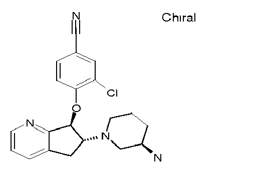 | HCl (2) | 0.68 (B) |

The compounds of the invention underwent pharmacological studies which demonstrated their ability to inhibit TRCP6. The method for testing the TRPC6 inhibitory activity of the compounds of the invention is as described in the patent application WO 2006/074802.

The IC₅₀ of the compounds of the invention are lower than 10 µM, demonstrating their value as therapeutically active substances. More specifically, the IC₅₀ values of the compounds described in table 1 are comprised between 0.001 µM and 1 µM. For example, compounds 8 and 63 display IC₅₀ of 1.20 x 10⁻⁸ and 8.11 x 10⁻⁷ µM, respectively.

The compounds according to the invention therefore display inhibition activity towards TRPC6.

The compounds of formula (I) are inhibitors of TRCP6, and are therefore useful for the prevention and treatment of fibrotic disorders, such as focal segmental glomerulosclerosis, skeletal muscle dysfunction, renal failure, atherosclerosis, heart failure, cancer (e.g. oesophageal cancer, breast cancer), chronic obstructive pulmonary disease, pain, pulmonary hypertension, ischemic stroke, myocardial infarction, inflammation or peripheral arterial occlusive disease.

The invention also relates to a medicament, comprising a compound of formula (I) as defined above, or an addition salt of said compound to a pharmaceutically acceptable salt, or an hydrate or solvate of said compound.

The invention also relates to compounds of formula (I) as drugs.

The compounds according to the invention can indeed be useful for the preparation of drugs, specifically of medicaments inhibiting TRCP6, in particular medicaments for the prevention and the treatment of fibrotic disorders, such as focal segmental glomerulosclerosis, skeletal muscle dysfunction, renal failure, atherosclerosis, heart failure, cancer (e.g. oesophageal cancer, breast cancer), chronic obstructive pulmonary disease, pain, pulmonary hypertension, ischemic stroke, myocardial infarction, inflammation or peripheral arterial occlusive disease.

The invention also relates to a pharmaceutical composition, comprising a compound of formula (I) as defined above, or an addition salt of said compound to a pharmaceutically acceptable salt, or an hydrate or solvate of said compound, as active principle, and at least one pharmaceutically acceptable excipient.

These pharmaceutical compositions comprise an effective dose of at least one compound according to the invention, or an addition salt thereof with a pharmaceutically acceptable salt, or an hydrate or solvate of the latter, and at least one pharmaceutically acceptable excipient.

Said excipients are chosen according to the pharmaceutical form and the administration route desired, among usual excipients known of one of skill in the art.

In the pharmaceutical compositions according to the invention for the oral, sublingual, sub-cutaneous, intramuscular, intra-venous, topical, local, intratracheal, intranasal, transdermal or rectal administration, the active principle of formula (I) above, its salt, solvate or hydrate, can be administered as a unitary dosage form, in blend with usual pharmaceutical excipients, to animals and human beings for the prevention or for the treatment of diseases mentioned above.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

As an example, a unitary dosage form for a compound according to the invention, in the form of a tablet, can comprise the following ingredients:

| | |
|---|---|
| Compound according to the invention | 50,0 mg |
| Mannitol | 223,75 mg |
| Croscarmellose sodique | 6,0 mg |
| Maize starch | 15,0 mg |
| Hydroxypropyl methylcellulose | 2,25 mg |
| Magnesium stearate | 3,0 mg |

The present invention, according to another of its aspects, also relates to a method for the treatment or prevention of the above pathologies, which comprises the administration to a patient of an effective dose of a compound according to the invention, or a salt with a pharmaceutically acceptable salt thereof, or an hydrate or a solvate thereof.

## Claims

1. Compound of formula (I) 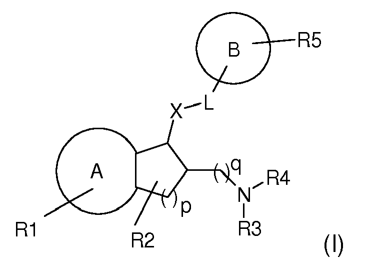 in which
A is a 6 to 10 membered aryl radical or a 5 to 10 membered heteroaryl radical, where the aryl and heteroaryl radical may be mono- or bicyclic, and the heteroaryl radical may comprise one or more heteroatoms selected from the group of nitrogen, oxygen and sulfur;
where one or more hydrogen atoms in said mono- or bicyclic aryl or heteroaryl radicals may be replaced by substituents R1 which are selected independently of one another from the group of H, F, Cl, Br, I, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₄-C₂₀)-cycloalkylalkyloxy, (C₁-C₁₀)-alkoxy, (C₁-C₁₀)-alkylthio, (C₆-C₁₄)-aryl, (C₂-C₁₃)-heteroaryl, -CN, -OH, -NR13R14, -C(O)R12, -SF₅, -S(O)ₙR12, -C(O)OR12, -C(O)NR13R14, -S(O)ₙNR13R14;
where two adjacent radicals R1 may also form a saturated or partly unsaturated (C₅-C₁₀)-cycloalkyl radical or a saturated or partly unsaturated (C₂-C₉)-cycloheteroalkyl radicals, where the cycloheteroalkyl radical may comprise 1, 2 or 3 nitrogen, 1 or 2 oxygen, 1 or 2 sulfur, 1 or 2 nitrogen and 1 oxygen or 1 sulfur atom;
where said alkyl, alkenyl, alkynyl, cycloalkyl, cycloalkylalkyl, cycloalkylalkyloxy, cycloheteroalkyl, alkoxy, and alkylthio radicals may be substituted independently of one another one or more times by F, OH or (C₁-C₁₀)-alkoxy;
B is a mono- or fused bicyclic radical selected from the group of
6 to 10 membered aryl radicals,
of 5 to 10 membered heteroaryl radicals,
of 3 to 10 membered cycloalkyl radicals,
of 9 to 14 membered cycloalkylaryl radicals,
of 8 to 14 membered cycloalkylheteroaryl radicals,
of 3 to 10 membered cycloheteroalkyl radicals,
of 9 to 14 membered cycloheteroalkylaryl radicals and
of 8 to 14 membered cycloheteroalkylheteroaryl radicals,
where the cycloalkyl or cycloheteroalkyl units may be saturated or partly unsaturated, and where the heterocyclic groups may comprise one or more heteroatoms selected from the group of nitrogen, oxygen and sulfur;
where one or more hydrogen atoms in the radicals B may be replaced by substituents R5 which are selected independently of one another from the group of (C₁-C₁₀)-alkyl radicals, of (C₂-C₁₀)-alkenyl radicals, of (C₂-C₁₀)-alkynyl radicals, of (C₁-C₁₀)-alkoxy radicals, of (C₁-C₁₀)-alkylthio radicals, of (C₃-C₁₄)-cycloalkyl radicals, of (C₄-C₂₀)-cycloalkylalkyl radicals, of (C₄-C₂₀)-cycloalkylalkyloxy, of (C₂-C₁₉)-cycloheteroalkyl radicals, of (C₃-C₁₉)-cycloheteroalkylalkyl radicals, of (C₃-C₁₁)-cycloalkyloxy radicals, of (C₂-C₁₁)-cycloheteroalkyloxy radicals, of (C₆-C₁₀)-aryl radicals, of (C₁-C₉)-heteroaryl radicals, of (C₉-C₁₄)-cycloalkylaryl radicals, of (C₅-C₁₃)-cycloalkylheteroaryl radicals, (C₇-C₁₃)-cycloheteroalkylaryl radicals, (C₄-C₁₂)-cycloheteroalkylheteroaryl radicals, where
the cycloalkyl and cycloheteroalkyl units may be saturated or partly unsaturated,
and where one or more hydrogen atoms in said radicals R5 may be replaced by further radicals which are selected independently of one another from the group of R11 radicals,
it is further possible for R5 to be one or more radicals which are selected independently of one another from the group of H, OH, (=O), NH₂, F, Cl, Br, I, CN, NO₂, -NR17R18, -NR16COR17, -NR16COOR17, -NR16CONR17R18, -NR16-S(O)₂-R17, -NR16-S(O)₂-NR17R18, -COOR16, -COR16; -CO(NR17R18), S(O)ₙR16, -S(O)₂NR17R18,
where R16, R17 and R18 independently of one another for a radical selected from the group of H, (C₂-C₁₉)-cycloheteroalkyl, (C₃-C₁₄)-cycloalkyl, (C₆-C₁₀)-aryl, (C₁-C₁₀)-alkyl radicals,
all of which may be substituted independently of one another by OH, (=O), F, Cl, Br, I, CN, NO₂, -NR13R14, -NR13COR12, -NR13COOR12, -NR12CONR13R14, -NR13-S(O)₂-R12, -NR12-S(O)₂-R13R14, -COOR12, -COR12; -CO(NR13R14),
-S(O)ₙR12, -S(O)₂NR13R14, (C₃-C₁₄)-cycloalkyl, (C₄-C₂₀)-cycloalkylalkyl, (C₂-C₁₉)-cycloheteroalkyl, (C₃-C₁₉)-cycloheteroalkylalkyl, (C₆-C₁₀)-aryl and (C₁-C₉)-heteroaryl,
and where R17 and R18 can form together with the nitrogen to which they are bonded a 4-7 membered, saturated, unsaturated or partly unsaturated heterocycle having 1 to 13 carbon atoms which may additionally comprise one or more heteroatoms from the list -O-, -S(O)ₙ-, =N- and -NR15-,
where the heterocycle formed may be substituted independently of one another one or more times by F, OH, (=O), NH₂, NH(C₁-C₄)alkyl, N((C₁-C₄)alkyl)₂, CN or (C₁-C₁₀)-alkoxy, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₂₀)-cycloheteroalkyl, (C₃-C₁₉)-cycloheteroalkylalkyl, each of which may in turn carry independently of one another one or more radicals F, OH, (=O), NH₂, NH(C₁-C₄)alkyl, N((C₁-C₄)alkyl)₂, CN or (C₁-C₁₀)-alkoxy;
L is a covalent bond or an alkylene bridge having 1 to 10 carbon atoms,
which may carry independently of one another one or more substituents from the group of radicals (C₁-Cₗ₁₀)-alkyl, (C₃-C₁₄)-cycloalkyl, (C₄-C₂₀)-cycloalkylalkyl radical, -COR12, -CO(NR13R14), S(O)ₙR12, -S(O)₂NR13R14, (=O) and F; where the alkyl, cycloalkyl and cycloalkyl radicals may be substituted one or more times by F;
X is a group -N(R6)-, -O-, -S(O)ₙ-, or alkylene having 1 to 5 carbon atoms, where R6 may be hydrogen or may be (C₁-C₁₀)-alkyl, (C₃-C₁₄)-cycloalkyl, (C₄-C₂₀)-cycloalkylalkyl radical, all of which may be substituted independently of one another one or more times by F, or R6 may be -COR12; -CO(NR13R14), S(O)ₙR12, -S(O)₂NR13R14;
R2 is absent or is one or more substituents which may be selected independently of one another from the group of F, (C₁-C₁₀)-alkyl and (C₁-C₁₀)-alkoxy radical, where the alkyl and alkoxy radicals may be substituted independently of one another one or more times by F;
R3 and R4 are independently of one another a hydrogen radical or a radical which is selected from the group of (C₁-C₁₀)-alkyl radicals, of (C₂-C₁₀)-alkenyl radicals, of (C₂-C₁₀)-alkynyl radicals, of (C₃-C₁₄)-cycloalkyl radicals, of (C₄-C₂₀)-cycloalkylalkyl radicals, of (C₂-C₁₉)-cycloheteroalkyl radicals, of (C₃-C₁₉)-cycloheteroalkylalkyl radicals, of (C₆-C₁₀)-aryl radicals, of (C₇-C₂₀)-arylalkyl radicals, of (C₁-C₉)-heteroaryl radicals, of (C₂-C₁₉)-heteroarylalkyl radicals, where
the radicals R3 and R4 may be substituted independently of one another one or more times by a radical from the group of OH, NH₂, (=O), F, CI, Br, I, CN, NO₂, -NR13R14, -NR13COR12, -NR13COOR12, -NR12CONR13R14, -NR13-S(O)₂-R12, -NR13-S(O)₂-NR13R14, -COOR12, -COR12; -CO(NR13R14), S(O)ₙR12, -S(O)₂R13R14, or
R3 and R4 form together with the nitrogen to which they are bonded a 4-10 membered, saturated, unsaturated or partly unsaturated heterocycle which may additionally comprise one or more heteroatoms from the list -O-, -S(O)ₙ-, =N- and -NR8-, where
the heterocyclic radicals may be substituted independently of one another one or more times by radicals selected from the group of R7 and R9, and where
the heterocyclic radicals may be bridged by a bond, by a saturated or unsaturated (C₁-C₁₀)-alkyl or (C₁-C₉)-heteroalkyl chain or by -NR15-, -O-, -S-, and where
the alkyl and heteroalkyl chains may also form a spirocyclic ring system with the ring system formed by R3 and R4, where the alkyl and heteroalkyl bridges may be substituted independently of one another one or more times by radicals selected from the group of R7 and R9,
and where
R8 in the group NR8 may form with the ring which R3 and R4 may form a further saturated, unsaturated or partly unsaturated heterocycle which may be substituted independently of one another one or more times by radicals selected from the group of R7 and R9, and may additionally comprise one or more heteroatoms from the list -O-, -S(O)ₙ-, -N= and -NR19-;
R7 are a (C₁-C₁₀)-alkyl radical or (C₁-C₁₄)-cycloalkyl radical, where the alkyl radical may be substituted independently of one another one or more times by R9;
R8 is an H, a (C₁-C₁₀)-alkyl radical or (C₁-C₁₄)-cycloalkyl radical, COR12, -CO(NR13R14), S(O)ₙR12, -S(O)₂NR13R14, where the alkyl radical may be substituted independently of one another one or more times by R10;
R9 is a radical selected from the group of OH, (=O), F, Cl, Br, I, CN, NO₂, -NR13R14, -NR13COR12, -NR13COOR12, -NR12CONR13R14, -NR13-S(O)₂-R12, -NR13-S(O)₂-NR13R14, -COOR12, -COR12, -CO(NR13R14), S(O)ₙR12, -S(O)₂NR13R14, (C₃-C₁₄)-cycloalkyl, (C₄-C₂₀)-cycloalkylalkyl, (C₁-C₁₀)-alkoxy, (C₂-C₁₉)-cycloheteroalkyl, (C₃-C₁₉)-cycloheteroalkylalkyl, (C₆-C₁₀)-aryl radicals, of (C₁-C₉)-heteroaryl radicals;
R10 is a radical selected from the group of F, OH, CN, (C₁-C₁₀)-alkoxy, (C₁-C₁₀)-alkylthio, NO₂, -NR13R14, -NR13COR12, -NR13COOR12, -NR13CONR13R14, -NR13-S(O)₂-R12, -NR12-S(O)₂-NR13R14, -COOR12, -COR12, -CO(NR13R14), S(O)ₙR12, -S(O)₂NR13R14;
R11 is a radical selected from the group of (C₁-C₁₀)-alkyl, (C₂-C₁₀)-alkenyl, (C₂-C₁₀)-alkynyl, (C₁-C₁₀)-alkoxy, (C₁-C₂₀)-alkylthio, (C₃-C₁₄)-cycloalkyl, (C₄-C₁₀)-cycloalkylalkyl, (C₂-C₁₃)-cycloheteroalkyl, (C₄-C₁₉)-cycloheteroalkylalkyl, (C₃-C₁₄)-cycloalkyloxy, (C₂-C₁₃)-cycloheteroalkyloxy,
all of which may be substituted independently of one another one or more times by R10;
(=O), Cl, Br, I and R10;
R12, R13 and R14 may independently of one another be H, (C₁-C₁₀)-alkyl, (C₂-C₁₀)-alkenyl, (C₂-C₁₀)-alkynyl, (C₃-C₁₄)-cycloalkyl, (C₄-C₁₀)-cycloalkylalkyl, (C₂-C₁₃)-cycloheteroalkyl, (C₃-C₁₉)-cycloheteroalkylalkyl, (C₆-C₁₀)-aryl, each of which may be substituted independently of one another one or more times by F, OH, (=O), NH₂, NH(C₁-C₄)alkyl, N((C₁-C₄)alkyl)₂, CN or (C₁-C₁₀)-alkoxy;
or where R13 and R14 may form together with the nitrogen to which they are bonded a 4-7 membered, saturated, unsaturated or partly unsaturated heterocycle having 1 to 13 carbon atoms, which may additionally comprise one or more heteroatoms from the list -O-, -S(O)ₙ-, =N- and -NR15-, where
the formed heterocycle may be substituted independently of one another one or more times by F, OH, (=O), NH₂, NH(C₁-C₄)alkyl, N((C₁-C₄)alkyl)₂, CN or (C₁-C₁₀)-alkoxy, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₂₀)-cycloheteroalkyl, (C₃-C₁₉)-cycloheteroalkylalkyl, each of which may in turn carry independently of one another one or more radicals F, OH, (=O), NH₂, NH(C₁-C₄)alkyl, N((C₁-C₄)alkyl)₂, CN or (C₁-C₁₀)-alkoxy;
R15 is a radical selected from the group of H, (C₁-C₁₀)-alkyl, (C₂-C₁₀)-alkenyl, (C₂-C₁₀)-alkynyl, (C₃-C₁₄)-cycloalkyl, (C₄-C₂₀)-cycloalkylalkyl, (C₂-C₁₃)-cycloheteroalkyl, (C₃-C₁₉)-cycloheteroalkylalkyl, each of which may be substituted independently of one another one or more times by F, OH, CN or (C₁-C₁₀)-alkoxy;
R19 is an H, a (C₁-C₁₀)-alkyl radical or (C₁-C₁₄)-cycloalkyl radical, COR12, -CO(NR13R14), S(O)ₙR12, -S(O)₂NR13R14, where the alkyl radical may be substituted independently of one another one or more times by R10;
and in which
n is 0, 1 or 2;
p is 1 or 2 and
q is 0 or 1,
in the form of a free base or of an addition salt with an acid, as well as in the form of an hydrate or of a solvate,
and in which :
i) in the case where A is phenyl, B is phenyl or benzodioxolanyl, X is -O- or -S-, L is a bond and R3 and R4 are H, (C₁-C₁₀)-alkyl, (C₃-C₁₄)-cycloalkyl, (C₇-C₂₀)-arylalkyl or R3 and R4 together are an unsubstituted pyrrolidinyl, morpholinyl, piperidinyl or piperazinyl radical or 4-methylpiperazinyl radical, at least one R5 radical which is not a (C₁-C₁₀)-alkyl, (C₁-C₁₀)-alkoxy, OH, CF₃, F, Cl, Br or I radical must be present,
ii) in the case where A is phenyl, X is -O-, -S- or -NH- and R3 and R4 are a (C₁-C₁₀)-alkyl, (C₃-C₁₄)-cycloalkyl or a (C₄-C₂₀)-cycloalkylalkyl radical, at least one R5 radical which is not an F, Cl, Br, I, (C₁-C₄)-alkyl, (C₁-C₄)-alkoxy, CF₃, OCF₃, CN, NO₂, NH₂, -NH((C₁-C₁₀)-alkyl), -N((C₁-C₁₀)-alkyl)₂, unsubstituted or substituted benzoyl or an unsubstituted or substituted phenyl-(CH₂)ᵣ-Y-(CH₂)ₛ- radical, with Y being a bond or an oxygen and r and s being 0 to 4, where r+s is not greater than 4, must be present.

2. Compound according to claim 1, of formula (II) : 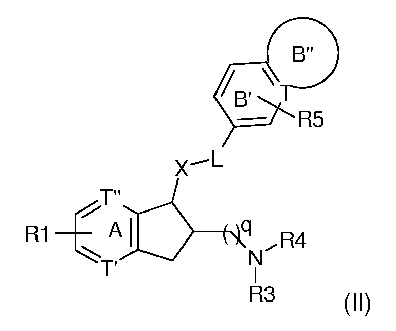 in which:
A is a 6 membered aryl radical or a 6 membered heteroaryl radical, where T' and T" are independently N or CR1;
where one or more hydrogen atoms in said aryl or heteroaryl radicals may be replaced by substituents R1 which are selected independently of one another from the group of H, F, CI, Br, I, (C₁-C₁₀)-alkyl-, (C₁-C₁₀)-alkoxy-, -CN, -OH, -S(O)ₙR12, -;
where said alkyl and alkoxy radicals may be substituted independently of one another one or more times by F and R12 is H or (C₁-C₁₀)-alkyl, optionally substituted one or more times by F;
B' is a 6 membered aryl radical or a 6 membered heteroaryl radical, where T is N or CR5;
B" is absent or is radical chosen from a (C₆-C₁₀)-aryl radical and a (C₃-C₆)-cycloheteroalkyl radical comprising one ore more heteroatoms chosen from O, N and S,
where at least one hydrogen atoms in the radicals B' and/or B" is replaced by substituents R5 which are selected independently of one another from the group of (C₁-C₁₀)-alkyl radicals, of (C₁-C₁₀)-alkoxy radicals, of (C₃-C₁₄)-cycloalkyl radicals, of (C₁-C₉)-heteroaryl radicals, where
the cycloalkyl units may be saturated or partly unsaturated, and where one or more hydrogen atoms in said radicals R5 may be replaced by further radicals which are selected independently of one another from the group of F, CI, Br and I,
it is further possible for R5 to be one or more radicals which are selected independently of one another from the group of OH, F, CI, Br, I, CN, NO₂, -COOR16, -CO(NR17R18),
where R16, R17 and R18 independently of one another for a radical selected from the group of H and (C₁-C₁₀)-alkyl radicals, which may be substituted independently of one another by F, Cl, Br, I,
L is a covalent bond or a methylene bridge;
X is a group -O- or -S(O)ₙ-;
R3 and R4 are independently of one another a hydrogen radical, a (C₁-C₁₀)-alkyl radical or a (C₃-C₁₉)-cycloheteroalkyl radical comprising at least one nitrogen atom, or
R3 and R4 form together with the nitrogen to which they are bonded a 4-7 membered, saturated mono-or bi-cycloheteroalkyl radical which may additionally comprise one or more -NR8- heteroatom, where
the cycloheteroalkyl radical may be substituted independently of one another one time by a radical-(CR20R21)ᵣ NR22R23, in which r is 0 or 1 and R20, R21, R22 and R23 are independently of one another H or a (C₁-C₁₀)-alkyl radical optionally substituted one or more times by F, Cl, Br and/or I,
R8 is an H or a (C₁-C₁₀)-alkyl radical;
and in which
n is 0, 1 or 2; and
q is 0 or 1,
in the form of a free base or of an addition salt with an acid, as well as in the form of an hydrate or of a solvate *(depending on the case).*

3. Compound according to claim 1 or 2, of formula (III): 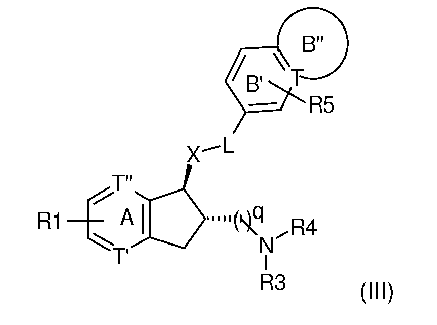 in which A, B', B", T, T', T", R1, R3, R4, R5, X, L and q are as defined in claim 2.
in the form of a free base or of an addition salt with an acid, as well as in the form of an hydrate or of a solvate.

4. Compound according to claim 1, chosen from the group:
(3R)-1-{(1R,2S)-5-chloro-1-[(2-chloro-5-fluorobenzyl)oxy]-2,3-dihydro-1H-inden-2-yl}piperidin-3-amine
(3S)-1-[(1R,2S)-5-chloro-1-1[2-fluoro-3-(trifluoromethyl)benzyl]oxyl-2,3-dihydro-1H-inden-2-yl]piperidin-3-amine
1-({(1S,2R)-1-[3-(trifluoromethoxy)phenoxy]-2,3-dihydro-1H-inden-2-yl}methyl)pyrrolidine
1-({(1S,2R)-1-[3-(trifluoromethoxy)phenoxy]-2,3-dihydro-1H-inden-2-yl}methyl)piperidine
1-[1-(2-cyclopentylphenoxy)-2,3-dihydro-1H-inden-2-yl]piperidin-4-amine
N-{(1-[(6-chloropyridin-3-yl)oxy]-2,3-dihydro-1H-inden-2-yl)-N-methylpiperidin-3-amine
(3R)-1-{(1R,2R)-1-[2-(2H-benzotriazol-2-yl)-4-methylphenoxy]-2,3-dihydro-1H-inden-2-yl}piperidin-3-amine
4-({(1R,2R)-2-[(3R)-3-aminopiperidin-1-yl]-2,3-dihydro-1H-inden-1-yl}oxy)-3-chlorobenzonitrile
(3R)-1-[(1R,2R)-4,6-dichloro-1-(4-fluorophenoxy)-2,3-dihydro-1H-inden-2-yl]piperidin-3-amine
4-({(1R,2R)-2-[(3R)-3-aminopiperidin-1-yl]-4,6-dichloro-2,3-dihydro-1H-inden-1-yl}oxy)-3-fluorobenzonitrile
4-({(1R,2R)-2-[(3R)-3-aminopiperidin-1-yl]-2,3-dihydro-1H-inden-1-yl}sulfanyl)benzonitrile
4-({(1R,2R)-2-[(3S)-3-aminopiperidin-1-yl]-2,3-dihydro-1H-inden-1-yl}oxy)benzonitrile
4-({(1R,2R)-2-[(3S)-3-aminopiperidin-1-yl]-2,3-dihydro-1H-inden-1-yl}oxy)-2-fluorobenzonitrile
4-({(1R,2R)-2-[(3S)-3-aminopiperidin-1-yl]-2,3-dihydro-1H-inden-1-yl}oxy)-3-fluorobenzonitrile
4-({(1R,2R)-2-[(3S)-3-aminopiperidin-1-yl]-2,3-dihydro-1H-inden-1-yl}oxy)-3-chlorobenzonitrile
4-({(1R,2R)-2-[(3S)-3-aminopiperidin-1-yl]-2,3-dihydro-1H-inden-1-yl}oxy)-2,3-difluorobenzonitrile
4-({(1R,2R)-2-[(3S)-3-aminopiperidin-1-yl]-2,3-dihydro-1H-inden-1-yl}oxy)-3-(trifluoromethyl)benzonitrile
(3S)-1-[(1R,2R)-1-(2-chloro-6-methylphenoxy)-2,3-dihydro-1H-inden-2-yl]piperidin-3-amine
(3S)-1-{(1R,2R)-1-[2-(2H-benzotriazol-2-yl)-4-methylphenoxy]-2,3-dihydro-1H-inden-2-yl}piperidin-3-amine
4-({(1R,2R)-2-[(3S)-3-aminopiperidin-1-yl]-2,3-dihydro-1H-inden-1-yl}oxy)-3-nitrobenzonitrile
2-({(1R,2R)-2-[(3S)-3-aminopiperidin-1-yl]-2,3-dihydro-1H-inden-1-yl}oxy)-5-bromobenzonitrile
2-({(1R,2R)-2-[(3S)-3-aminopiperidin-1-yl]-2,3-dihydro-1H-inden-1-yl}oxy)-5-chlorobenzonitrile
(3S)-1-[(1R,2R)-1-(2-bromo-4,6-difluorophenoxy)-2,3-dihydro-1H-inden-2-yl]piperidin-3-amine
4-({(1R,2R)-2-[(3S)-3-aminopiperidin-1-yl]-2,3-dihydro-1H-inden-1-yl}oxy)-3-bromobenzonitrile
1-[(1R,2R)-1-(2-bromophenoxy)-2,3-dihydro-1H-inden-2-yl]-1,4-diazepane
1-[(1R,2R)-1-(2-chloro-6-methylphenoxy)-2,3-dihydro-1H-inden-2-yl]-1,4-diazepane
1-[(1R,2R)-1-(2-bromo-4-fluorophenoxy)-2,3-dihydro-1H-inden-2-yl]-1,4-diazepane
1-[(1R,2R)-1-(2,3,6-trifluorophenoxy)-2,3-dihydro-1H-inden-2-yl]-1,4-diazepane
1-[(1R,2R)-1-(2,4,6-trifluorophenoxy)-2,3-dihydro-1H-inden-2-yl]-1,4-diazepane
1-[(1R,2R)-1-(2-bromo-4,6-difluorophenoxy)-2,3-dihydro-1H-inden-2-yl]-1,4-diazepane
(3R)-1-[(1R,2R)-1-(2,4-dichlorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)benzonitrile
4-({(1R,2R)-2-[(3R)-3-aminopiperidin-1-yl]-2,3-dihydro-1H-inden-1-yl}oxy)-3-fluorobenzonitrile
4-({(1R,2R)-2-[(3R)-3-aminopiperidin-1-yl]-2,3-dihydro-1H-inden-1-yl}oxy)-2-chlorobenzonitrile
4-({(1R,2R)-2-[(3R)-3-aminopiperidin-1-yl]-2,3-dihydro-1H-inden-1-yl}oxy)-2,3-difluorobenzonitrile
4-({(1R,2R)-2-[(3R)-3-aminopiperidin-1-yl]-2,3-dihydro-1H-inden-1-yl}oxy)-3-(trifluoromethyl)benzonitrile
4-({(1R,2R)-2-[(3R)-3-aminopiperidin-1-yl]-2,3-dihydro-1H-inden-1-yl}oxy)-3-methoxybenzonitrile
(3R)-1-[(1R,2R)-1-(4-chloro-2-methylphenoxy)-2,3-dihydro-1H-inden-2-yl]piperidin-3-amine
(3R)-1-[(1R,2R)-1-(2,4-difluorophenoxy)-2,3-dihydro-1H-inden-2-yl]piperidin-3-amine
(3R)-1-[(1R,2R)-1-(4-bromo-3-fluorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)-3-nitrobenzonitrile
(3R)-1-[(1R,2R)-1-(4-fluoro-2-methylphenoxy)-2,3-dihydro-1H-inden-2-yl]piperidin-3-amine
2-({(1R,2R)-2-[(3R)-3-aminopiperidin-1-yl]-2,3-dihydro-1H-inden-1-yl}oxy)-5-bromobenzonitrile
2-({(1R,2R)-2-[(3R)-3-aminopiperidin-1-yl]-2,3-dihydro-1H-inden-1-yl}oxy)-5-chlorobenzonitrile
5-({(1R,2R)-2-[(3R)-3-aminopiperidin-1-yl]-2,3-dihydro-1H-inden-1-yl}oxy)-1,3-benzoxathiol-2-one
2-({(1R,2R)-2-[(3R)-3-aminopiperidin-1-yl]-2,3-dihydro-1H-inden-1-yl}oxy)-6-fluorobenzonitrile
(3R)-1-{(1R,2R)-1-[4-bromo-2-(1H-pyrazol-3-yl)phenoxy]-2,3-dihydro-1H-inden-2-yl}piperidin-3-amine
4-({(1R,2R)-2-[(3R)-3-aminopiperidin-1-yl]-2,3-dihydro-1H-inden-1-yl}oxy)-3-ethoxybenzonitrile
2-({(1R,2R)-2-[(3R)-3-aminopiperidin-1-yl]-2,3-dihydro-1H-inden-1-yl}oxy)benzonitrile
(3R)-1-[(1R,2R)-1-(4-bromophenoxy)-2,3-dihydro-1H-inden-2-yl]piperidin-3-amine
(3R)-1-[(1R,2R)-1-(4-fluorophenoxy)-2,3-dihydro-1H-inden-2-yl]piperidin-3-amine
(3R)-1-[(1R,2R)-1-(2-bromo-4-chlorophenoxy)-2,3-dihydro-1H-inden-2-yl]piperidin-3-amine
2-({(1R,2R)-2-[(3R)-3-aminopiperidin-1-yl]-2,3-dihydro-1H-inden-1-yl}oxy)-5-chlorobenzamide
6-({(1R,2R)-2-[(3R)-3-aminopiperidin-1-yl]-2,3-dihydro-1H-iden-1-yl}oxy)-3,4-dihydroquinolin-2(1H)-one
(3R)-1-{(1R,2R)-1-[4-methyl-2-(1H-pyrazol-3-yl)phenoxy]-2,3-dihydro-1H-inden-2-yl}piperidin-3-amine
3-({(1R,2R)-2-[(3R)-3-aminopiperidin-1-yl]-2,3-dihydro-1H-inden-1-yl}oxy)-N,N-dimethylnaphthalene-2-carboxamide
2-({(1R,2R)-2-[(3R)-3-aminopiperidin-1-yl]-2,3-dihydro-1H-inden-1-yl}oxy)-4-bromobenzonitrile
2-({(1R,2R)-2-[(3S)-3-aminopiperidin-1-yl]-2,3-dihydro-1H-inden-1-yl}oxy)-6-fluorobenzonitrile
4-({(1R,2R)-2-[(3R)-3-aminopyrrolidin-1-yl]-2,3-dihydro-1H-inden-1-yl}oxy)benzonitrile
4-({(1R,2R)-2-[(3R)-3-aminopyrrolidin-1-yl]-2,3-dihydro-1H-inden-1-yl}oxy)-3-fluorobenzonitrile
4-({(1R,2R)-2-[(3R)-3-aminopyrrolidin-1-yl]-2,3-dihydro-1H-inden-1-yl}oxy)-3-chlorobenzonitrile
(3R)-1-{(1R,2R)-1-[2-(2H-benzotriazol-2-yl)-4-methylphenoxy]-2,3-dihydro-1H-inden-2-yl}pyrrolidin-3-amine
4-({(1R,2R)-2-[(3R)-3-aminopyrrolidin-1-yl]-2,3-dihydro-1H-inden-1-yl}oxy)-3-nitrobenzonitrile
(3R)-1-[(1R,2R)-1-(2-bromo-4-fluorophenoxy)-2,3-dihydro-1H-inden-2-yl]piperidin-3-amine
(3R)-1-{(1R,2R)-1-[4-chloro-2-(1H-pyrazol-3-yl)phenoxy]-2,3-dihydro-1H-inden-2-yl}piperidin-3-amine
(3R)-1-{(1R,2R)-1-[4-chloro-2-(1,2-oxazol-5-yl)phenoxy]-2,3-dihydro-1H-inden-2-yl}piperidin-3-amine
(3R)-1-[(1R,2R)-1-(2-bromo-4,6-difluorophenoxy)-2,3-dihydro-1H-inden-2-yl]piperidin-3-amine
(3R)-1-[(1R,2R)-1-(4-chloro-2-fluorophenoxy)-2,3-dihydro-1H-inden-2-yl]piperidin-3-amine
4-({(1R,2R)-2-[(3R)-3-aminopiperidin-1-yl]-2,3-dihydro-1H-inden-1-yl}oxy)-3-bromobenzonitrile
(3R)-1-{(1R,2R)-1-[4-bromo-2-(1,2-oxazol-5-yl)phenoxy]-2,3-dihydro-1H-inden-2-yl}piperidin-3-amine
4-({(1R,2R)-2-[(3S)-3-aminopyrrolidin-1-yl]-2,3-dihydro-1H-inden-1-yl}oxy)benzonitrile
4-({(1R,2R)-2-[(3S)-3-aminopyrrolidin-1-yl]-2,3-dihydro-1H-inden-1-yl}oxy)-2-fluorobenzonitrile
4-({(1R,2R)-2-[(3S)-3-aminopyrrolidin-1-yl]-2,3-dihydro-1H-inden-1-yl}oxy)-3-fluorobenzonitrile
4-({(1R,2R)-2-[(3S)-3-aminopyrrolidin-1-yl]-2,3-dihydro-1H-inden-1-yl}oxy)-3-chlorobenzonitrile
4-({(1R,2R)-2-[(3S)-3-aminopyrrolidin-1-yl]-2,3-dihydro-1H-inden-1-yl}oxy)-2,3-difluorobenzonitrile
1-[(1R,2R)-1-(2-chloro-6-fluoro-3-methylphenoxy)-2,3-dihydro-1H-inden-2-yl]-1,4-diazepane
(3S)-1-{(1R,2R)-1-[2-(2H-benzotriazol-2-yl)-4-methylphenoxy]-2,3-dihydro-1H-inden-2-yl}pyrrolidin-3-amine
4-({(1R,2R)-2-[(3S)-3-aminopyrrolidin-1-yl]-2,3-dihydro-1H-inden-1-yl}oxy)-3-nitrobenzonitrile
2-({-2-[(3S)-3-aminopyrrolidin-1-yl]-2,3-dihydro-1H-inden-1-yl}oxy)-5-bromobenzonitrile
2-({(1R,2R)-2-[(3S)-3-aminopyrrolidin-1-yl]-2,3-dihydro-1H-inden-1-yl}oxy)-5-chlorobenzonitrile
4-({(1S,2S)-2-[(3R)-3-aminopiperidin-1-yl]-4-methyl-2,3-dihydro-1H-inden-1-yl}oxy)-3-chlorobenzonitrile
4-({(1R,2R)-2-[3-(methylamino)pyrrolidin-1-yl]-2,3-dihydro-1H-inden-1-yl}oxy)benzonitrile
3-fluoro-4-({(1R,2R)-2-[3-(methylamino)pyrrolidin-1-yl]-2,3-dihydro-1H-inden-1-yl}oxy)benzonitrile
3-chloro-4-({(1R,2R)-2-[3-(methylamino)pyrrolidin-1-yl]-2,3-dihydro-1H-inden-1-yl}oxy)benzonitrile
1-{(1R,2R)-1-[2-(2H-benzotriazol-2-yl)-4-methylphenoxy]-2,3-dihydro-1H-inden-2-yl}-N-methylpyrrolidin-3-amine
1-{(3R)-1-[(1R,2R)-1-(4-nitrophenoxy)-2,3-dihydro-1H-inden-2-yl]pyrrolidin-3-yl}methanamine
1-{(3R)-1-[(1R,2R)-1-(4-bromophenoxy)-2,3-dihydro-1H-inden-2-yl]pyrrolidin-3-yl}methanamine
1-{(3R)-1-[(1R,2R)-1-(2-chloro-4-fluorophenoxy)-2,3-dihydro-1H-inden-2-yl]pyrrolidin-3-yl}methanamine
1-{(3R)-1-[(1R,2R)-1-(2-chloro-4-nitrophenoxy)-2,3-dihydro-1H-inden-2-yl]pyrrolidin-3-yl}methanamine
2-({(1R,2R)-2-[(3R)-3-(aminomethyl)pyrrolidin-1-yl]-2,3-dihydro-1H-inden-1-yl}oxy)benzonitrile
1-{(3R)-1-[(1R,2R)-1-(2-bromo-4-chlorophenoxy)-2,3-dihydro-1H-inden-2-yl]pyrrolidin-3-yl}methanamine
2-({(1R,2R)-2-[(3R)-3-(aminomethyl)pyrrolidin-1-yl]-2,3-dihydro-1H-inden-1-yl}oxy)-5-chlorobenzonitrile
methyl 4-({(1R,2R)-2-[(3R)-3-(aminomethyl)pyrrolidin-1-yl]-2,3-dihydro-1H-inden-1-yl}oxy)-3-chlorobenzoate
4-({(1R,2R)-2-[(3R)-3-(aminomethyl)pyrrolidin-1-yl]-2,3-dihydro-1H-inden-1-yl}oxy)-3-chlorobenzonitrile
1-{(3R)-1-[(1R,2R)-1-(4-bromo-2-chlorophenoxy)-2,3-dihydro-1H-inden-2-yl]pyrrolidin-3-yl}methanamine
1-[(3R)-1-{(1R,2R)-1-[4-bromo-2-(1H-pyrazol-3-yl)phenoxy]-2,3-dihydro-1H-inden-2-yl]pyrrolidin-3-yl]methanamine
2-({(1R,2R)-2-[(3R)-3-(aminomethyl)pyrrolidin-1-yl]-2,3-dihydro-1H-inden-1-yl}oxy)-5-bromobenzonitrile
4-({(1R,2R)-2-[(3R)-3-(aminomethyl)pyrrolidin-1-yl]-2,3-dihydro-1H-inden-1-yl}oxy)-3-bromobenzonitrile
1-[(3R)-1-{(1R,2R)-1-[4-chloro-2-(1H-pyrazol-3-yl)phenoxy]-2,3-dihydro-1H-inden-2-yl}pyrrolidin-3-yl]methanamine
1-[(3R)-1-{(1R,2R)-1-[4-methyl-2-(1H-pyrazol-3-yl)phenoxy]-2,3-dihydro-1H-inden-2-yl}pyrrolidin-3-yl]methanamine
1-{(3R)-1-[(1R,2R)-1-(2,4-dichlorophenoxy)-2,3-dihydro-1H-inden-2-yl]pyrrolidin-3-yl}methanamine
1-{(3R)-1-[(1R,2R)-1-(4-chlorophenoxy)-2,3-dihydro-1H-inden-2-yl]pyrrolidin-3-yl}methanamine
4-({(1R,2R)-2-[(3R)-3-(aminomethyl)pyrrolidin-1-yl]-2,3-dihydro-1H-iden-1-yl}oxy)-2-fluorobenzonitrile
4-({(1R,2R)-2-[(3R)-3-(aminomethyl)pyrrolidin-1-yl]-2,3-dihydro-1H-inden-1-yl}oxy)-3-methoxybenzonitrile
4-({(1R,2R)-2-[(3R)-3-(aminomethyl)pyrrolidin-1-yl]-2,3-dihydro-1H-inden-1-yl}oxy)-2-chlorobenzonitrile
4-({(1R,2R)-2-[(3R)-3-(aminomethyl)pyrrolidin-1-yl]-2,3-dihydro-1H-inden-1-yl}oxy)-2,6-difluorobenzonitrile
2-({(1R,2R)-2-[(3R)-3-(aminomethyl)pyrrolidin-1-yl]-2,3-dihydro-1H-inden-1-yl}oxy)-6-fluorobenzonitrile
4-({(1R,2R)-2-[(3R)-3-(aminomethyl)pyrrolidin-1-yl]-2,3-dihydro-1H-inden-1-yl}oxy)-3-fluorobenzonitrile
4-({(1R,2R)-2-[(3R)-3-(aminomethyl)pyrrolidin-1-yl]-2,3-dihydro-1H-inden-1-yl}oxy)-2,3-difluorobenzonitrile
4-({(1R,2R)-2-[(3R)-3-(aminomethyl)pyrrolidin-1-yl]-2,3-dihydro-1H-inden-1-yl}oxy)-3-(trifluoromethyl)benzonitrile
1-{(3S)-1-[(1R,2R)-1-(4-nitrophenoxy)-2,3-dihydro-1H-inden-2-yl]pyrrolidin-3-yl}methanamine
1-{(3S)-1-[(1R,2R)-1-(2-chloro-4-nitrophenoxy)-2,3-dihydro-1H-inden-2-yl]pyrrolidin-3-yl}methanamine
2-({(1R,2R)-2-[(3S)-3-(aminomethyl)pyrrolidin-1-yl]-2,3-dihydro-1H-inden-1-yl}oxy)-5-chlorobenzonitrile
4-({(1R,2R)-2-[(3S)-3-(aminomethyl)pyrrolidin-1-yl]-2,3-dihydro-1H-inden-1-yl}oxy)-3-chlorobenzonitrile
2-({(1R,2R)-2-[(3S)-3-(aminomethyl)pyrrolidin-1-yl]-2,3-dihydro-1H-inden-1-yl}oxy)-5-bromobenzonitrile
1-{(3S)-1-[(1R,2R)-1-(4-chlorophenoxy)-2,3-dihydro-1H-inden-2-yl]pyrrolidin-3-yl}methanamine
4-({(1R,2R)-2-[(3S)-3-(aminomethyl)pyrrolidin-1-yl]-2,3-dihydro-1H-inden-1-yl}oxy)-2-fluorobenzonitrile
4-({(1R,2R)-2-[(3S)-3-(aminomethyl)pyrrolidin-1-yl]-2,3-dihydro-1H-inden-1-yl}oxy)-2-chlorobenzonitrile
3-chloro-4-({(1R,2R)-2-[4-(dimethylamino)piperidin-1-yl]-2,3-dihydro-1H-inden-1-yl}oxy)benzonitrile
4-({(1R,2R)-2-[(3R)-3-aminopiperidin-1-yl]-6-methoxy-2,3-dihydro-1H-inden-1-yl}oxy)-3-chlorobenzonitrile
(2R,3R)-2-[(3R)-3-aminopiperidin-1-yl]-3-(2-chloro-4-cyanophenoxy)-2,3-dihydro-1H-indene-5-carbonitrile
(1 R,2R)-2-[(3R)-3-aminopiperidin-1-yl]-1-(2-chloro-4-cyanophenoxy)-2,3-dihydro-1H-indene-5-carbonitrile
4-{[(1R,2R)-2-[(3R)-3-aminopiperidin-1-yl]-6-(methylsulfonyl)-2,3-dihydro-1H-inden-1-yl]oxy}-3-chlorobenzonitrile
4-({(1R,2R)-2-[(3R)-3-aminopiperidin-1-yl]-5-fluoro-2,3-dihydro-1H-inden-1-yl}oxy)-3-chlorobenzonitrile
4-({(1R,2R)-2-[3-(2-aminopropan-2-yl)azetidin-1-yl]-2,3-dihydro-1H-inden-1-yl}oxy)-3-chlorobenzonitrile
3-chloro-4-({(1R,2R)-2-[(1 S,4S)-2,5-diazabicyclo[2.2.1]hept-2-yl]-2,3-dihydro-1H-inden-1-yl}oxy)benzonitrile
methyl 4-({(1R,2R)-2-[(3R)-3-aminopiperidin-1-yl]-2,3-dihydro-1H-inden-1-yl}oxy)-3-chlorobenzoate
3-chloro-4-({(1R,2R)-2-[(3R)-3-(methylamino)piperidin-1-yl]-2,3-dihydro-1H-inden-1-yl}oxy)benzonitrile
1-{(1R,2R)-1-[2-(2H-benzotriazol-2-yl)-4-methylphenoxy]-2,3-dihydro-1H-inden-2-yl}-N-methylpyrrolidin-3-amine
4-({(1R,2R)-2-[(3R)-3-aminopiperidin-1-yl]-6-hydroxy-2,3-dihydro-1H-inden-1-yl}oxy)-3-chlorobenzonitrile
6-({(1R,2R)-2-[(3R)-3-aminopiperidin-1-yl]-6-methoxy-2,3-dihydro-1H-inden-1-yl}oxy)-3,4-dihydroquinolin-2(1H)-one
6-({(1R,2R)-2-[(3R)-3-aminopiperidin-1-yl]-2,3-dihydro-1H-inden-1-yl}oxy)-7-chloro-3,4-dihydroquinolin-2(1H)-one
4-({(1R,2R)-2-[(3R)-3-aminopiperidin-1-yl]-5-bromo-2,3-dihydro-1H-inden-1-yl}oxy)-3-chlorobenzonitrile
4-({(1R,2R)-2-[(3R)-3-aminopiperidin-1-yl]-4,6-dichloro-2,3-dihydro-1H-inden-1-yl}oxy)-3-chlorobenzonitrile
6-({(1R,2R)-2-[(3R)-3-aminopiperidin-1-yl]-5-chloro-6-methoxy-2,3-dihydro-1H-inden-1-yl}oxy)-3,4-dihydroquinolin-2(1H)-one
6-{[(1R,2R)-2-[(3R)-3-aminopiperidin-1-yl]-6-(trifluoromethyl)-2,3-dihydro-1H-inden-1-yl]oxy}-3,4-dihydroquinolin-2(1H)-one
4-({(5R,6R)-6-[(3R)-3-aminopiperidin-1-yl]-6,7-dihydro-5H-cyclopenta[b]pyridin-5-yl}oxy)-3-chlorobenzonitrile
4-({(6R,7S)-6-[(3R)-3-aminopiperidin-1-yl]-6,7-dihydro-5H-cyclopenta[b]pyridin-7-yl}oxy)-3-chlorobenzonitrile;
in the form of a free base or of an addition salt with an acid, as well as in the form of an hydrate or of a solvate.

5. Medicament, comprising a compound of formula (I) according to claims 1 to 4, or an addition salt of said compound to a pharmaceutically acceptable salt, or an hydrate or solvate of said compound.

6. Pharmaceutical composition, comprising a compound of formula (I) according to claims 1 to 4, or an addition salt of said compound to a pharmaceutically acceptable salt, or an hydrate or solvate of said compound, and at least one pharmaceutically acceptable excipient.

7. Compound of formula (I) according to claims 1 to 4 for the prevention and treatment of fibrotic disorders, such as focal segmental glomerulosclerosis, skeletal muscle dysfunction, renal failure, atherosclerosis, heart failure, cancer (e.g. oesophageal cancer, breast cancer), chronic obstructive pulmonary disease, pain, pulmonary hypertension, ischemic stroke, myocardial infarction, inflammation or peripheral arterial occlusive disease.
